# EUROPEAN PATENT SPECIFICATION

(11) **EP 2 176 666 B1**
(45) Date of publication and mention of the grant of the patent: **19.02.2014**
(21) Application number: 08784983.2
(22) Date of filing: 23.07.2008
(51) Int. Cl.: G01N 33/68, G01N 33/50

(54) **NOVEL AS160-LIKE PROTEIN, TEST SYSTEMS, METHODS AND USES INVOLVING IT FOR THE IDENTIFICATION OF DIABETES TYPE 2 THERAPEUTICS**
NEUES AS160-PROTEIN, TESTSYSTEME, VERFAHREN UND VERWENDUNGEN BEI DER IDENTIFIZIERUNG VON THERAPEUTIKA GEGEN TYP-2-DIABETES
NOUVELLE PROTÉINE DE TYPE AS160, SYSTÈMES DE TESTS, PROCÉDÉS ET UTILISATIONS L'IMPLIQUANT POUR L'IDENTIFICATION D'AGENTS THÉRAPEUTIQUES POUR LE DIABÈTE DE TYPE 2

(30) Priority: 01.08.2007 EP 07015086
(43) Date of publication of application: 21.04.2010
(60) Divisional application: 10185772.0
(73) Proprietor: SANOFI, 75008 Paris (FR)
(72) Inventor: TENNAGELS, Norbert, 65926 Frankfurt am Main (DE); BAUS, Daniela, 65926 Frankfurt am Main (DE); HEERMEIER, Kathrin, 65926 Frankfurt am Main (DE); DITTRICH, Werner, 65926 Frankfurt am Main (DE)
(74) Representative: Körner, Kathrin
(86) International application number: PCT/EP2008/006024
(87) International publication number: WO 2009/015808

(56) References cited:
- WO-A-02/099049
- WO-A-2005/016962
- WO-A-2006/099005
- US-A1- 2005 014 194
- US-A1- 2006 160 076
- ROACH WILLIAM G ET AL: "Substrate specificity and effect on GLUT4 translocation of the Rab GTPase-activating protein Tbc1d1." THE BIOCHEMICAL JOURNAL 15 APR 2007, vol. 403, no. 2, 15 April 2007 (2007-04-15), pages 353-358, XP002460390 ISSN: 1470-8728
- BAI LI ET AL: "Dissecting multiple steps of GLUT4 trafficking and identifying the sites of insulin action" CELL METABOLISM, vol. 5, no. 1, January 2007 (2007-01), pages 47-57 URL, XP008086296 ISSN: 1550-4131
- CARTEE GREGORY D ET AL: "Role of Akt substrate of 160 kDa in insulin-stimulated and contraction-stimulated glucose transport." APPLIED PHYSIOLOGY, NUTRITION, AND METABOLISM = PHYSIOLOGIE APPLIQUÉE, NUTRITION ET MÉTABOLISME JUN 2007, vol. 32, no. 3, June 2007 (2007-06), pages 557-566, XP008086288 ISSN: 1715-5312
- KANE SUSAN ET AL: "A method to identify serine kinase substrates. Akt phosphorylates a novel adipocyte protein with a Rab GTPase-activating protein (GAP) domain" JOURNAL OF BIOLOGICAL CHEMISTRY, vol. 277, no. 25, 21 June 2002 (2002-06-21), pages 22115-22118, XP002460258 ISSN: 0021-9258 cited in the application
- H. SANO: 'Insulin-stimulated Phosphorylation of a Rab GTPase-activating Protein Regulates GLUT4 Translocation' JOURNAL OF BIOLOGICAL CHEMISTRY vol. 278, no. 17, 18 April 2003, pages 14599 - 14602, XP055007185 DOI: 10.1074/jbc.C300063200 ISSN: 0021-9258 -& DATABASE UNIPROT [Online] 10 October 2002 'RecName: Full=TBC1 domain family member 4; AltName: Full=Akt substrate of 160 kDa; Short=AS160;' Retrieved from EBI, accession no. UNIPROT:O60343 Database accession no. O60343

## Description

The present invention relates to novel AKT substrate 160kDA-like protein (AS 160-like protein); a method of identifying a substance altering glucose uptake of a cell comprising contacting a test system comprising novel AKT substrate 160kDa-like protein (AS160-like protein) with a test substance, and identifying a test substance as a substance altering glucose uptake of a cell by detecting a signal indicative for altered glucose uptake of a cell; a test system comprising a gene coding for the AKT substrate 160 kDa-like protein (AS160-like protein) and an inducible promoter providing for controllable expression of the gene; the use of the test system for the identification of a substance improving glucose uptake into a cell; and the use of AS160-like protein in a model for type 2 diabetes.

Diabetes mellitus is a metabolic disorder characterized by hyperglycemia and other signs, as distinct from a single illness or condition. The World Health Organization recognizes three main forms of diabetes: type 1, type 2, and gestational diabetes (occurring during pregnancy), which have similar signs, symptoms, and consequences, but different causes and population distributions. Ultimately, all forms are due to the beta cells of the pancreas being unable to produce sufficient insulin to prevent hyperglycemia.

Type 1 is usually due to autoimmune destruction of the pancreatic beta cells which produce insulin. Type 2 is characterized by tissue-wide insulin resistance, particularly of insulin-sensitive tissues comprising adipose tissue, liver and skeletal muscle, and varies widely; it sometimes progresses to loss of beta cell function. Gestational diabetes is similar to type 2-diabetes, in that it involves insulin resistance caused by hormones of pregnancy.

Types 1 and 2 are incurable chronic conditions, but have been treatable and are usually managed with a combination of dietary treatment and medicaments including insulin supplementation.

Diabetes can cause many complications such as hypoglycemia, ketoacidosis or nonketotic hyperosmolar coma. Serious long-term complications include cardiovascular disease (doubled risk), chronic renal failure (diabetic nephropathy is the main cause of dialysis in developed world adults), retinal damage (which can lead to blindness and is the most significant cause of adult blindness in the non-elderly in the developed world), nerve damage (of several kinds), and microvascular damage, which may cause erectile dysfunction (impotence) and poor healing. Poor healing of wounds, particularly of the feet, can lead to gangrene which can require amputation - the leading cause of non-traumatic amputation in adults in the developed world.

Because insulin is the principal hormone that regulates uptake of glucose into most cells from the blood (primarily muscle and adipocytes), deficiency of insulin or the insensitivity of its receptors plays a central role in all forms of diabetes mellitus. Insulin is released into the blood by β- cells in the pancreas in response to rising levels of blood glucose (e.g., after a meal). Insulin enables most body cells (about 2/3 is the usual estimate, including muscle cells and adipose tissue) to absorb glucose from the blood.

Type 2 diabetes mellitus is due to a combination of defective insulin secretion and insulin resistance or reduced insulin sensitivity of insulin-sensitive tissues, particularly adipose tissue, liver and skeletal muscle. In the early stage the predominant abnormality is reduced insulin sensitivity, characterized by elevated levels of insulin in the blood. At this stage, hyperglycemia can be reversed by a variety of measures and medications that improve insulin sensitivity or reduce glucose production by the liver, but as the disease progresses, the impairment of insulin secretion worsens, and therapeutic replacement of insulin often becomes necessary.

Usually, type 2 diabetes is first treated by attempts to change physical activity, the diet (generally to decrease carbohydrate intake), and weight loss. The usual next step, if necessary, is treatment with oral antidiabetic drugs. As insulin production is initially only moderately impaired in type 2 diabetics, oral medication can still be used to improve insulin production, to regulate inappropriate release of glucose by the liver and to substantially attenuate insulin resistance.

Adequate treatment of diabetes in early the stage, particularly improvement insulin sensitivity of adipose tissue, liver and skeletal muscle, may protract, retard and/or prevent progression of the disease.

Accordingly, a first object of the invention was to better understand the molecular background involved in the glucose metabolism, thus helping to better search for new potential drugs improving insulin sensitivity of cells, particularly of cells of skeletal muscle, adipose tissue and/or liver, which are the main insulin-sensitive tissues.

The present invention in its broadest sense is defined by the appended claims.

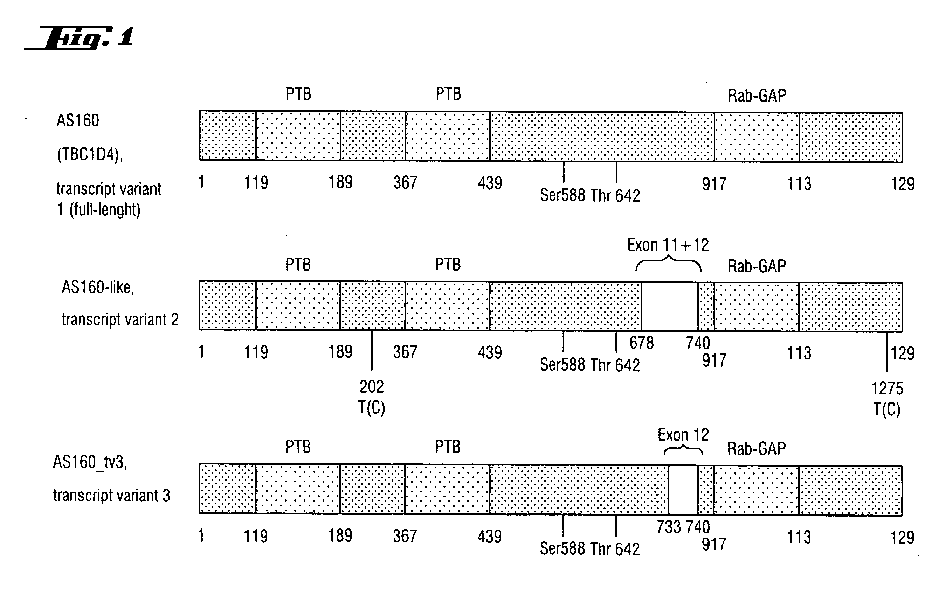

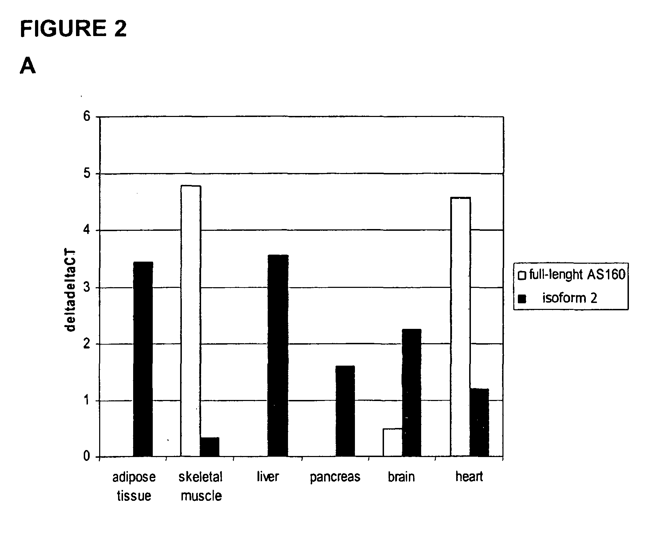

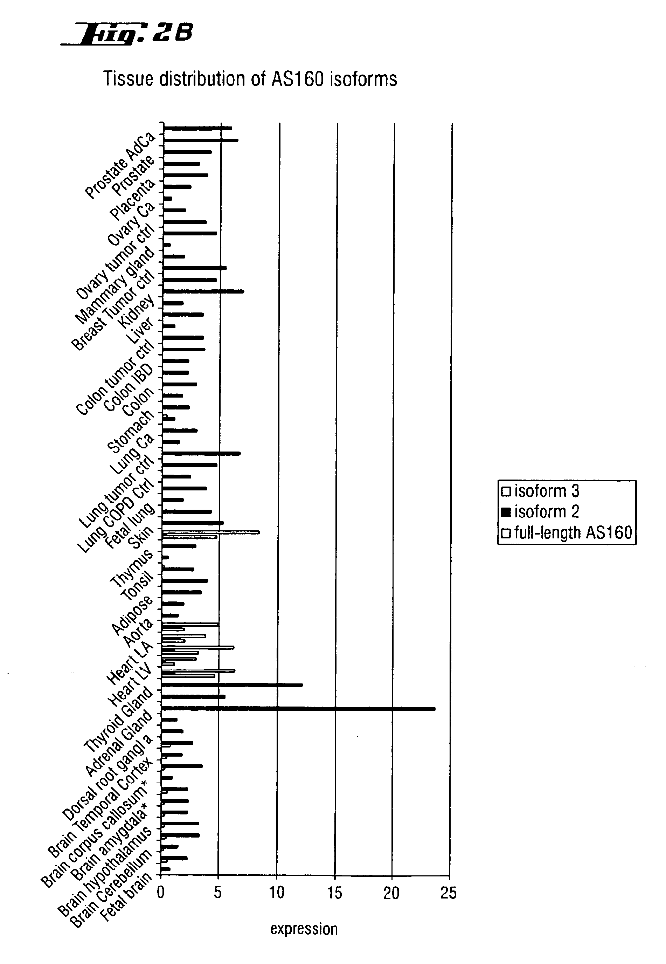

Surprisingly, two novel isoforms (isoforms 2 and 3) of AKT substrate 160kDa (AS160, also referred to as Tbc1 D4 or AS160, isoform 1) have now been identified by the inventors. In the following, the term "AS160-like protein", refers to either or both novel isoforms of AS160 i.e. to isoforms 2 and/or 3. AS160, isoforms AS160, isoform 2 is expressed in the six main insulin-sensitive tissues, i.e. adipose tissue, liver, skeletal muscle, heart, brain and pancreatic tissue (see Figure 2 A, B). In contrast to this, the isoform 1 of AS160, is predominantly detected in skeletal muscle and heart. The third isoform (AS160, isoform 3), lacking exon 12 of AS160 (Figure 1) is also mainly expressed in heart and skeletal muscle. (Figure 2C). As an ineffective insulin action is a hallmark of type 2 diabetes, AS160-like protein provides a novel target to study the molecular basis of insulin-resistance in insulin-sensitive tissues. Additionally, AS160-like protein may be used to identify substances which could improve glucose uptake, and therefore insulin sensitivity, in the relevant tissues.

Components of the intracellular signal transduction pathway involving AS160-like protein were identified which allows for studying interaction of a substance with the respective signal transduction pathway at different levels. It could be shown that insulin-stimulated signal transduction relating to AS160-like protein involves phosphorylation of AS160 and AKT, involvement of PI3K (PI3-kinase) and MEKK/ERK kinases. Unexpectedly, the inventors found that overexpression of AS160-like results in enhanced translocation of GLUT4 to the plasma membrane and increase in glucose uptake.

They also found that a test system involving AS160-like protein may be used to study glucose uptake of cells under high glucose conditions. This may be particularly important for a diabetes model or for the identification of a suitable therapeutic for the treatment and/or prevention of diabetes, as this disease is characterized by increased levels of glucose in the blood. Accordingly, testing a substance capable of altering, particularly improving, glucose uptake under this condition (high glucose) might be beneficial for the identification of a new therapeutic.

Accordingly, AS160-like protein may be used in a method of identifying a substance altering, particularly increasing, glucose uptake of a cell and, therefore, having a potential for the treatment or prevention of type 2 diabetes.

WO2006/099005 describes a method for identifying agents that modulate the GTPase activity of AS160. The amino acid sequence of AS160 described in W02006/099005 corresponds to isoform 1 of the AKT substrate 160kDA protein.

Sano et al., J. Biol. Chem. 278: 14599-14602 (2003) is a scientific publication describing that the insulin-stimulated phosphorylation of a Rab GTPase-activating protein regulates GLUT4 translocation. The authors refer to a polypeptide consisting of a chain of 1298 amino acid residues, while isoform 2 of the AKT substrate 160kDA protein consists of a chain of 1235 amino acid residues.

Therefore, the present invention provides in a first aspect a method of identifying a substance altering glucose uptake and/or GLUT4 translocation of a cell comprising
(a) contacting a test system comprising AKT substrate 160kDa-like protein (AS160-like protein) with a test substance, and
(b) identifying a test substance as a substance altering glucose uptake and/or GLUT4 translocation of a cell by detecting a signal indicative for altered glucose uptake of a cell, wherein the AS160-like protein
   (i) is encoded by the nucleic add having SEQ ID NO: 1,
   (ii) is encoded by a nucleic add encoding a protein consisting of the SEQ ID NO: 3 and short C- and/or N-terminal sequences of at most 30, 25, 20, 15, 10, 9, 8, 7, 6, 5, 4, 3, 2, or 1 amino adds homologous or heterologous to the protein,
   (iii) is a protein having the amino add sequence according to SEQ ID NO: 3,
   (iv) has 99,5% or more sequence homology with SEQ ID NO: 3, or
   (v) is a protein having an amino add sequence according to the sequence NM_014832 but lacking the amino adds 678 to 740 when compared to this AS 160 amino add sequence,
   wherein the test system is in a cell of an insulin-dependent tissue.

"Altering glucose uptake of a cell" in the context of the present invention means a change, either increase or decrease, of glucose uptake of a cell. Preferably the glucose uptake of a cell is increased.

In the context of the present invention, the glucose uptake of a cell is altered, i.e. decreased or increased in comparison to a control, if the glucose uptake of a cell contacted with the (test) substance is significantly lower or higher, respectively, than that of the control (e.g. the same cell not contacted with the (test) substance). The person skilled in the art knows statistical procedures to assess whether two values are significantly different from each other such as Student's t-test or chi-squared test (see also Examples for suitable test methods).

In a preferred embodiment of increased glucose uptake, the glucose uptake of a cell amounts to at least 110 %, preferably to at least 125 %, more preferably to at least 150 %, 160 %, 170 %, 180 % or 190 %, still more preferably to at least 200 % and most preferably to at least 300 % of the control.

As detailed above, for the treatment or prevention of type 2 diabetes it is particularly important to modify glucose uptake of an insulin-sensitive tissue. Accordingly, it is preferred that the method allows for the identification of a substance altering, preferably increasing, the glucose uptake in at least one, preferably at least two, more preferably at least three or at least four or at least five or at least six insulin-sensitive tissue(s). Examples of insulin-sensitive tissues include, without limitation, adipose tissue, liver, skeletal muscle, pancreatic tissue, myocardium, vascular smooth muscle and active mammary gland.

However, the six main insulin-sensitive tissues are adipose tissue, liver skeletal muscle, heart, brain and pancreatic tissue. Accordingly, the substance preferably alters, more preferably increases, glucose uptake in at least one, two, three, four, five or six or more of these tissues, i.e. adipose tissue, skeletal muscle, heart, brain, pancreatic tissue and/or liver.

If glucose uptake is altered, preferably increased, in one tissue, this could be, for example, any of: adipose tissue, liver, heart, brain, pancreatic tissue or skeletal muscle.

If glucose uptake is altered, preferably increased, in two tissues, this could be, for example,
adipose tissue and liver;
adipose tissue and skeletal muscle; or
liver and skeletal muscle, or any other combination of two of the six main insulin-sensitive tissues as listed above.

If glucose uptake is altered, preferably increased, in three tissues, this could be, for example, adipose tissue, liver and skeletal muscle or any other combination of three of the above-listed six main insulin-sensitive tissues.

If glucose uptake is altered, preferably increased in four tissues, this could be, for example, adipose tissue, liver, skeletal muscle and brain or any other combination of four of the above-listed six main insulin-sensitive tissues.

If glucose uptake is altered, preferably increased in five tissues, this could be, for example, adipose tissue, liver, skeletal muscle, brain and heart, or any other combination of five of the above-listed six main insulin-sensitive tissues.

The main cell types present in adipose tissue, liver, heart, brain, pancreatic tissue and skeletal muscle are adipocytes, hepatocytes, heart muscle cells, neuronal cells pancreatic cells, beta cells and skeletal muscle cells, respectively. Accordingly, the substance preferably alters, more preferably increases, glucose uptake in at least one, two or three of these cell types, i.e. adipocytes, hepatocytes and/or skeletal muscle cells.

If glucose uptake is altered, preferably increased, in one cell type, this could be, for example in adipocytes, hepatocytes, heart muscle cells, neuronal cells pancreatic cells, beta cells or skeletal muscle cells.

If glucose uptake is altered, preferably increased, in two cell types, this could be, for example,
adipocytes and hepatocytes;
adipocytes and skeletal muscle cells; or
hepatocytes and skeletal muscle cells or any other combination of the main cell types present in the six main insulin-sensitive tissues as listed above.

If glucose uptake is altered, preferably increased, in three cell types, this could be, for example, adipocytes, hepatocytes and skeletal muscle cells or any other combination of the above listed main cell types present in the six main insulin-sensitive tissues as listed above.

If glucose uptake is altered, preferably increased in four, five or more of the main cell types as listed above for the six main insulin-sensitive tissues, this can be any combination of four, five or more of those cell types.

As detailed above, the method of the invention involves a test system comprising isoform 2 of AS160 protein. As described herein, isoform 2 of AS160 protein is derived from AKT substrate 160kDa (AS160), wherein in isoform 2 in comparison to AS160, the sequence encoded by exons 11 and 12 in AS160 is missing (see also Figure 1). Isoform 3 of AS160, is derived from AKT substrate 160kDa (AS160), wherein in comparison to AS160, the sequence of isoform 3 lacks exon 12.

Additionally, further mutations may be present as detailed below.

AS160 (AKT substrate 160kDa; NM_014832 (EMBL)) was originally identified as a substrate of the protein kinase AKT in 3T3 adipocytes (Kane et al., 2002). Additional studies demonstrated that AS160 also plays a role in skeletal muscles of mice, rats and humans. Insulin, contraction or AICAR (5-aminoimidozole-4-carboxamide 1 β-D-ribonucleoside, cAMP-dependent protein kinase (cAMPK) activator) increase phosphorylation of AS160 on two sites (Ser 588 and Thr 642) which lie in characteristic motifs predicted for AKT phosphorylation (RXRXXS/T) (Kane et al., supra). A prominent feature of AS160 is the presence of a GTPase activating domain for Rab proteins. These small G-proteins are required for membrane trafficking. In this context, recent data provide evidence that AS160 links signals downstream of AKT with the insulin-stimulated translocation of GLUT4 (Sano et al., 2003). AS160 activation is reduced in patients with type II diabetes, resulting in an impaired GLUT4 translocation (Karlsson et al., 2005b). Overexpression of full-length AS160 in adipocytes did not alter the basal or insulin-stimulated surface-to-total distribution of GLUT4 indicating that the amount of AS160 seems not to be rate-limiting (Zeigerer et al., 2004; Sano et al., 2003). Experiments with mutant AS160 (containing 4 mutated phosphorylation sites) showed that GLUT4 translocation is markedly reduced (Sano et al., 2003). Additionally, a functional GAP (GTPase-activating protein) domain of AS160 is required for GLUT4 translocation.

AS 160, isoforms 2: In the gene encoding novel isoform 2 of AS160, exons 11 and 12 are missing in comparison to the full-length AS160 gene (see Examples 1 and 2). In case of the human gene, the nucleotides encoding amino acids 678 to 740 are missing in comparison to the human full-length AS160 as defined by the sequence NM_014832 (see EMBL data base). Furthermore, two mismatches were identified at positions nt 606 (silent) and nt 3827 (Ala → Val). In addition, the clone of Example 2 contained a 3 bp deletion (nt 2594-2596) that was also found in human placenta cDNA but not in human brain cDNA. For the expression clone of the isoform lacking exons 11 and 12, which was used in the Examples, the deleted 3bp sequence was reintroduced to resemble more closely the full length sequence of NM_014832 (EMBL). The resulting DNA sequence encoding isoform 2 of AS160 (SEQ ID NO: 1) is given in the following:
DNA Sequence encoding isoform 2 of AS160 (SEQ ID NO: 1)

The translated amino acid sequence via EditSeq (Lasergene) (SEQ ID NO: 3) of novel isoform 2 of AS160 is given in the following:

As 160, isoform 3: In the gene encoding novel isoform 3 of AS160, exon 12 is deleted compared to full length AS160 gene. This exon corresponds to amino acids 733 to 740 with respect to NM_014832 (see EMBL data base). The resulting DNA sequence encoding isoform 3 of AS160 (SEQ ID NO: 22) is given in the following:
DNA Sequence encoding isoform3 of AS160 (SEQ ID No: 22)

The translated amino acid sequence via EditSeq (Lasergene) (SEQ ID NO:23) of novel isoform3 of AS 160 is given in the following:

The term "AS 160-like protein" refers to both or either of isoforms 2 or 3 of AS160. The term "isoform 2 of AS160" refers to a protein, whose gene is a naturally occurring variant of the AS160 gene in which exons 11 and 12 are deleted. The term "isoforms 3 of AS160" refers to a protein, whose gene is a naturally occurring variant of the AS160 gene, in which exon 12 is lacking in comparison to the full-length AS160.

Additionally, short mismatches may be present in the AS 160-like protein, if the sequence of AS160-like protein is compared to that of AS160. A short mismatch is intended to relate to an addition, deletion, or substitution of up to 5 adjacent amino acids, preferably up to 4, more preferably up to 3, still more preferably up to 2, most preferably up to 1 adjacent amino acid. Within the naturally occurring AS160-like protein there may by up to 5 additions, deletions, and/or substitutions, preferably up to 4, more preferably up to 3, 2 or 1 additions, deletions, and/or substitutions In comparison to AS160.

Exemplary deletions and substitutions are those mentioned above, namely a deletion of 1 amino acid at a position corresponding to that encoded by nt 2594-2596 of the human AS160 gene or a substitution (e.g. Ala → Val) at a position corresponding to that encoded by nt 3827-3829 of the human AS160 gene. It is noted that it is intend that the AS160-iike protein or the nucleic acid coding the same may also be derived from species other than human including, but not limited to mammal, such as monkey, rodent (e.g. mouse or rat), dog, cat, cattle, pig, horse, sheep, goat or to avian, such as chicken or to amphibian, such as frog; however, the mammalian or human amino acid and nucleic acid sequences are preferred. The positions in AS160 or AS160-like protein sequences of species other than human corresponding to positions of the human sequences specified herein may be determined by sequence alignments as known to the skilled practitioner.

In one embodiment of the invention AS160-like protein consists of the sequence of SEQ ID NO: 3 and C- and/or N-terminal additions, such as short C- and/or N-terminal sequences of at most 30, 25, 20, 15, 10, 9, 8, 7, 6, 5, 4, 3, 2, or 1 amino acids heterologous to the protein as defined below.

The feature "AS160-like protein" relates to, e.g.,
1) a protein encoded by a nucleic acid comprising or having 90% or more, preferably 95% or more, more preferably 97% or more, more preferably 99% or more sequence homology with a nucleic acid sequence according to SEQ ID NO:1 or SEQ ID NO: 22
2) a protein encoded by a nucleic acid comprising or having the nucleic acid sequence according to SEQ ID NO:1 or SEQ ID NO: 22,or
3) a protein encoded by a nucleic acid hybridizing with a nucleic acid having the nucleic acid sequence according to SEQ ID NO:1 or SEQ ID NO: 22 under conditions of stringency, or
4) a protein having the amino acid sequence according to SEQ ID NO:3 or SEQ ID NO: 23, or
5) a protein having an amino acid sequence of 95% or more, preferably 97% or more, more preferably 98% or more, more preferably 99% or more and preferably 99,5% or more sequence homology with SEQ ID NO:3 or SEQ ID NO: 23,
6) a protein having an amino acid sequence of known AS160 (preferably, of human AS 160 and more preferably of AS 160 according to the sequence NM_014832 (see EMBL data base) but lacking amino acids 600 to 800, preferably lacking the amino acids 650 to 770, more preferably lacking the amino acids 670 to 750, more preferably the amino acids 675 to 745 and most preferably lacking the amino acids 678 to 740 when compared to this AS 160 amino acid sequence, or
7) a protein having an amino acid sequence of known AS160 (preferably, of human AS 160 and more preferably of AS 160 according to the sequence NM_014832 (see EMBL data base) but lacking the amino acids encoded by exon 12, preferably lacking amino acids 733 to 740.
8) a functional fragment or a functional derivative of one of the AS 160-like proteins as defined above under 1 to 7,
the above proteins preferably having at least one of the functional characteristics of AS 160-like protein as specified above and below.

A fragment is a protein that carries one or more end-terminal (n- and/or c-terminal) or internal deletions of one, two or more amino acids, when compared to the full-length protein. A functional fragment of a protein is any fragment of this protein having at least one and preferably two or more of the functional characteristics of the full-length protein.

The term derivative of a protein comprises any type of modification of the protein in comparison to the naturally-occurring form (in the context of present application especially in comparison to AS 160-like according to SEQ ID NO:3), that is not a deletion. A functional derivative of a protein is any derivative of this protein having at least one and preferably two or more of the functional characteristics of the unmodified protein.

The present description also comprises functional derivatives of fragments of AS160-like protein.

The determination of homology of amino acid or nucleic acid sequences can e.g. be made by use of the program GAP (GCG Program Package, Genetic Computer Group 1991) or any other of the programs known in the art.
Isolated polynucleotides and oligonucleotides can be used for hybridizing at different conditions of stringency.

A nucleic acid molecule can hybridise to another nucleic acid molecule when the single stranded forms of both molecules can anneal under suitable reaction ("annealing" or hybridisation) conditions (depending on temperature and ionic strength of the surrounding medium) to form a new double stranded nucleic acid molecule. Hybridisation requires that the two annealing nucleic acid molecules comprise complementary sequences. Depending on the selected annealing conditions, the stringency conditions, mismatches between the bases are possible without preventing double strand formation.

The term stringency describes reaction conditions that influence the specificity of hybridisation or annealing of two single stranded nucleic acid molecules. Stringency, and thus specificity of a reaction depends, inter alia, of the temperature and buffer-conditions used for a reaction: Stringency, and thus specificity, can e.g. be increased by increasing the reaction temperature and/or lowering the ion strength of the reaction-buffer. Suitable conditions of stringency for the hybridisation of nucleic acids depend on the length, the type of nucleic acid and their degree of complementarity. The variables are known in the state of the art. The greater the degree of similarity or homology between two annealing nucleotide sequences, the greater the melting temperature for hybridisation products of nucleic acids with those sequences. The relative stability of nucleic acid hybridisation is dependent according to the type of the single stranded nucleic acids forming the double strand:
RNA:RNA>DNA:RNA>DNA:DNA. For hybridisation products of greater than 100 nucleotides in length, equations for calculating the melting temperature are known in the art. For shorter hybridisation products (e.g. oligonucleotides) the calculation of the melting temperature is dependent on the length, wherein mismatches become more important.

Conditions of low stringency (and thus low reaction and hybridisation specificity) exist for example, if a hybridisation is performed at room temperature in 2xSSC-solution. Conditions of high stringency comprise e.g. a hybridisation reaction at 68°C in 0,1xSSC and 0,1% SDS solution.

In the context of present invention the term "hybridising under conditions of stringency" refers to conditions for the performance of the hybridisation reaction and the following washing procedure, at which nucleotide sequences with a certain complementarity typically remain hybridised. The choice of such conditions for a given set of nucleic acids lies within the skill of the average artisan, and suitable protocols can be found in well known literature for standard methods like, for example, "Current Protocols in Molecular Biology", John Wiley & Sons, N.Y. (1989), 6.3.1 to 6.3.6.

Hybridisation under conditions of stringency within the different aspects of present invention is preferably understood to be:
Hybridising a labelled probe with a nucleic acid sample to be analysed at 65°C, or in the case of oligonucleotide probes, at 5°C below the annealing or melting temperature of the duplex consisting of oligonucleotide and sample (annealing and melting temperature are in the following understood to be synonyms) over night in 50mM Tris pH 7,5, 1M NaCl, 1% SDS, 10% Dextran Sulfate, 0,5 mg/ml denatured salmon or herring sperm DNA.
Washing for 10 minutes in 2xSSC at room temperature.
Washing for 30 minutes in 1xSSC/0,1%SDS at 65°C (or in the case of oligonucleotides: 5°C below the annealing temperature).
Washing for 30 minutes in 0,1 xSSC/0,1 %SDS at 65°C (or in the case of oligonucleotides: 5°C below the annealing temperature).

In one embodiment, the AS160-like protein is encoded by a nucleic acid consisting of the sequence of SEQ ID NO: 1 or relating to a nucleic acid encoding a protein consisting of the sequence encoded by SEQ ID NO: 1 and short C- and/or N-terminal sequences of at most 30, 25, 20,15, 10, 9, 8, 7, 6, 5, 4, 3, 2, or 1 amino acids homologous or heterologous to the protein. These sequences may results from the genetic manipulations, e.g. the use of particular restriction sites, or may be needed for the purification of the protein, e.g. tags such as His-tag, Strep-tag, Arg-tag, c-myc-tag or Flag-Tag.

The feature "heterologous amino acid" or "amino acid heterologous to the protein" refers to any amino acid which is different from that amino acid located adjacent to a naturally occurring As160-like protein, AS160 protein or a splice variant thereof. Therefore, the AS160-like protein encompassing at least one heterologous amino acid refers to a protein which is different from any naturally occurring AS160 protein or splice variant thereof.

A functional characteristic of novel AS 160-like protein can be any characteristic of the AS 160-like protein as specified herein. Examples of such functional characteristics encompass, but are not limited to, e.g.: its tissue distribution, its implication in insulin-stimulated signal transduction modulation such as modulation and especially stimulation of insulin-stimulated glucose uptake, a modulation and especially stimulation of the phosphorylation of AS160 and AKT, a modulation and especially stimulation of activity of PI3K (PI3-kinase) and MEKK/ERK kinases, modulation and especially stimulation of the translocation of GLUT4 to the plasma membrane, the interaction of AS160-like protein with other proteins, e.g. the interaction of AS160-like protein with GLUT4 and any other of its functional characteristics, especially as depicted in the context of this application and the experimental results given below.

According to the invention, the test system is in a cell of an insulin-dependent tissue. A cell-based system is advantageous, because it allows for easy amplification of the test system by propagating the cells and cellular mechanisms, e.g. signal transduction components downstream of insulin or downstream or upstream of AS160-like protein, as these may be used in order to detect a signal indicative for altered glucose uptake of a cell.

Examples of cells suitable in the context of the present invention include without limitation L6 cells, 3T3 adipocytes, HEK 293, 745-A, A-431, atrial myocytes, BxPC3, C5N, Caco-2, Capan-1, CC531, CFPAC, CHO, CHO K1, COS-1, COS-7, CV-1, EAHY, EAHY 926, F98, GH3, GP&envAM12, H-295 R, H-4-II-E, HACAT, HACAT A131, HEK, HEL, HeLa, Hep G2, High Five, Hs 766T, HT29, HUV-EC R24, HUV-EC-C, IEC 17, IEC 18, Jurkat, K 562, KARPAS-299, L 929, LIN 175, MAt-LYLU, MCF-7, MNEL, MRC-5, MT4, N64, NCTC 2544, NDCK II, Neuro 2A, NIH 3T3, NT2/D1, P19, primary neuronal cells, primary dendritic cells, primary human or mammalian myoblasts, primary adipocytes, primary keratinocytes, SF9, SK-UT-1, ST, SW 480, SWU-2 OS, U-373, U-937, rhabdomyosarcoma (RD) and Y-1. Other suitable cells are known to the one of skill in the art.

However, preferably the test system is in a cell of an insulin-dependent tissue such as adipose tissue, liver, skeletal muscle, myocardium, vascular smooth muscle and active mammary gland, preferably skeletal muscle, adipose or liver, since these are the main insulin-dependent cell types in the mammalian body. Particularly suitable cells include skeletal muscle cell, adipocyte and/or hepatocyte, as these cells might best reflect the response to a substance in the tissues relevant in type 2 diabetes.

Cells that are cultured directly from an animal or a person are known as primary cells. With the exception of some cell lines derived from tumours, most primary cell cultures have limited lifespan. After a certain number of population doublings cells undergo the process of senescence and stop dividing, while generally retaining viability.

An established or immortalised cell line has acquired the ability to proliferate indefinitely either through random mutation or deliberate modification, such as artificial expression of the telomerase gene. There are numerous well established cell lines representative of particular cell types and it is within the knowledge of the skilled person to select a suitable cell line.

Accordingly, in a preferred embodiment of the invention the cell is a cell line. A cell line is a population of cells propagated in culture that are derived from, and therefore genetically identical to, a single common ancestor cell. Preferred cell lines are L6 cells (see Examples), HEK 293 cells (primary human embryonic kidney), 3T3 cells (murine embryonic fibroblasts), CHO cells (Chinese hamster ovary), COS-7 cells (African green monkey cell line), HeLa cells (human epithelioid cervical carcinoma), JURKAT cells (human T-cell leukaemia), BHK 21 cell (hamster normal kidney, fibroblast), and MCF-7 cells (human breast cancer).

Preferred cell lines of skeletal muscle, liver or adipose tissue include without being limited thereto:
Skeletal muscle: L6 cells (see also Examples), C2C12 (mouse), preferably DSM ACC2853 (see below).
Adipose tissue: 3T3 adipocytes, brown adipocyte cell line HIB-1 B), line F44-2A, those disclosed in US patent NO: 6,071,747.
Liver: BNL CL.2 (mouse, BALB/c), BNL SV A.8 (mouse), RLC-18 (rat) WRL 68.

A particularly preferred cell line encompassing a gene coding for isoforms 2 of AS 160 under the control of a tetracycline-responsive promoter system (L6-GLUT4myc-tetR-AS160-like) was deposited under the Budapest Treaty on the International Recognition of the Deposit of Microorganisms for the Purposes of Patent Procedure at the "Deutsche Sammlung von Mikroorganismen und Zellkulturen GmbH" (DSMZ), Inhoffenstraße 7 B, 38124 Braunschweig, GERMANY under the accession number DSM ACC2853 (referred to as L6-GLUT4myc-tetR-AS160-like) was deposited on June 20, 2007.

In order to screen for AS160-like protein, and especially isoforms 2 of AS160-mediated effects, the results obtained with the afore-mentioned cell line may be compared to those obtained with the same cell line, but lacking a introduction of a gene coding for AS160-like protein (L6-GLUT4myc-tetR) which was also deposited under the Budapest Treaty on the International Recognition of the Deposit of Microorganisms for the Purposes of Patent Procedure at the "Deutsche Sammlung von Mikroorganismen und Zellkulturen GmbH" (DSMZ), Inhoffenstraße 7 B, 38124 Braunschweig, GERMANY under the accession number DSM ACC2852 (referred to as L6-GLUT4myc-tetR) on June 20, 2007.

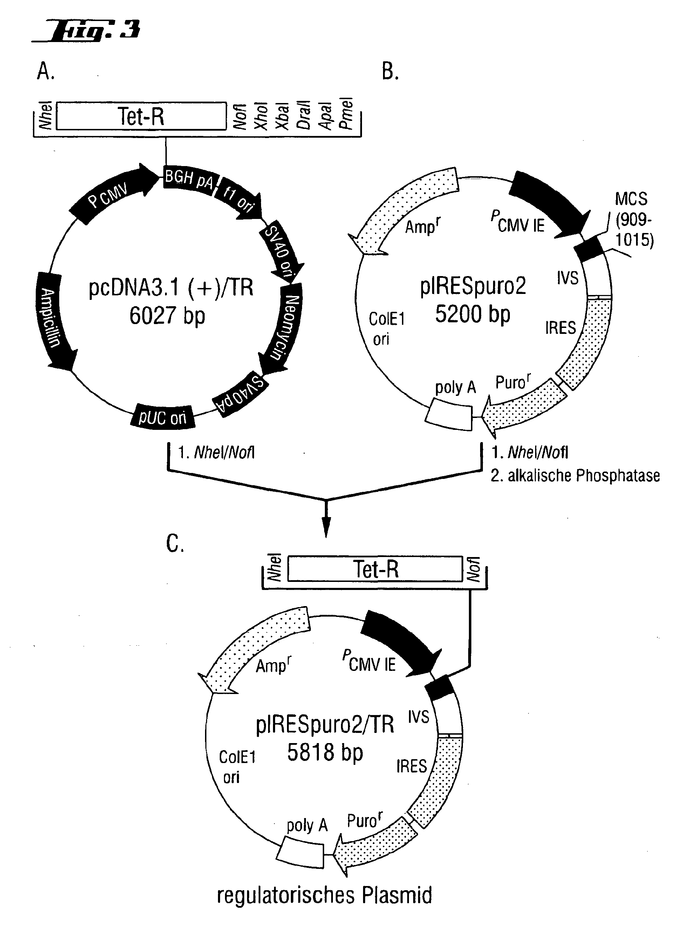

Both cell lines (L6-GLUT4myc-tetR-AS160-like and L6-GLUT4myc-tetR) have been prepared as detailed in Example 2. Briefly summarized, the tetracycline-repressor (TR) was isolated from pCDNA3.1 (+)/TR (Invitrogen), cloned into the Nhel and Notl sites of pIRESpuro2 as shown in Figure 3 to obtain pIRESpuro2/TR which was transfected into L6 cells (rat skeletal muscle cells) stably expressing GLUT4myc (L6-GLUT4myc, described in Wang et al. 1998). For L6-GLUT4myc-tetR-AS160-like cells, pCDNA5 vector (Invitrogen) containing the AS160, isoforms 2 gene was additionally introduced into the cells.

Analogously, a cell line expressing isoforms 3 of AS160-like protein can be prepared using similar procedures according to standard protocols known to the person skilled in the art.

For cultivation, cells may be grown and maintained at an appropriate temperature and gas mixture (typically, 37°C, 5% CO₂) in a cell incubator. Culture conditions vary widely for each cell type, and variation of conditions for a particular cell type can result in different phenotypes being expressed. Aside from temperature and gas mixture, the most commonly varied factor in culture systems is the growth medium. Recipes for growth media can vary in pH, glucose concentration, growth factors, and the presence of other nutrient components. Antibiotics can also be added to the growth media. Amongst the common manipulations carried out on culture cells are media changes and passaging cells. However, selection of suitable conditions is known to the skilled person.

The cell or cell line may be genetically engineered to include the test system of the invention. The test system may be located in a transient or stable transfected cell or cell line. The procedure for introducing a transgene into a recipient cell is called transfection. Transfection with DNA yields stable as well as unstable (transient) cells or cell lines. Transient cell lines reflect the survival of the transfected DNA in extrachromosomal form; stable cell lines result from the integration into the genome.

The transgenes can be introduced into the cells by a variety of means known to those knowledgeable in the art, and adapted to each cell type. Recombinant DNA cloning techniques well known in the art for introducing and expressing a nucleic acid molecule can be used to introduce and express the transgenes. Cells can be transfected using any appropriate means, including viral vectors, chemical transfectants, electroporation, calcium phosphate co-precipitation and direct diffusion of DNA. A suitable method for introducing a tests system into a recipient cell is detailed in Example 2 and may be adapted to the respective recipient cell.

As used herein, vectors are agents that transport the transgene into the cell and may include appropriate transcriptional and translational control signals such as a promoter. Vectors can be plasmid, viral or others known in the art. The promoter can be inducible or constitutive, general or cell specific, nuclear or cytoplasmic specific promoter. Selection of promoters, vectors and other elements is a matter of routine design within the level of ordinary skill in the art. Many such elements are described in the literature and are available through commercial suppliers. Usually, the method of transfer includes the transfer of a selectable marker to the cells. Suitable promoters and vectors are disclosed in the Examples and the present description.

In general, a cell line is transfected by any of the means mentioned above, wherein the transgene is operatively linked to a selectable marker. Following transfection cells are grown e.g. for some days in enriched media and then switched to selective media. Transfected cells exhibit resistance to the selection and are able to grow, whereas non-transfected cells die in general. Examples for selective markers include puromycin, zeocin, neomycin (neo) and hygromycin B, which confer resistance to puromycin, zeocin, aminoglycoside G-418 and hygromycin, respectively, and/or any of the selective markers used in the Examples. However, other selection methods known to the skilled person may be also suitable.

In the step b) of the method of the present invention a test substance is identified as a substance altering glucose uptake of a cell by detecting a signal indicative for altered glucose uptake of a cell. The signal may be any suitable signal which is indicative for altered glucose uptake of a cell; however, the signal may by any component or part of the insulin-stimulated signal transduction relating to AS160-like protein. Particularly, it may be the degree of phosphorylation of AS160 or AKT, activity of PI3K (PI3-kinase) or MEKK/ERK kinases, translocation of GLUT4 to the plasma membrane or increase in glucose uptake of a cell.

Suitable methods for measuring the aforementioned components of the AS160-like protein signal transduction pathway are known in the art and are also detailed in the Examples.

Preferably, the detectable signal is the amount of AS160-like protein (isoforms 2 and/or 3) expressed in a cell, phosphorylated AKT, phosphorylated AS160-like protein, GLUT4 translocation to the plasma membrane, GLUT4 distribution in a cell or glucose uptake by a cell. As could be shown in the examples, all these signals correlate with the glucose uptake of a cell, preferably a cell of an insulin-sensitive tissue.

The detectable signal may be the amount of AS160-like protein in a cell, as the amount of this protein is indicative for glucose uptake. If the amount of this protein is increased, the glucose uptake of a cell, particularly an insulin-sensitive cell, is increased, too. Methods of determining the amount of a particular protein are known to the skilled person and include e.g. Western blotting and detection with specific antibodies, which may be carried out as detailed in the Examples. A specific antibody is also provided in the Examples. Alternatively, an anti-AS160-like monoclonal or polyclonal antibody may be produced in accordance with the knowledge of the skilled person and detected by enzyme-linked immunosorbent assay (ELISA), radioimmunoassay (RIA), and fluorescence activated cell sorting (FACS).

A variety of other techniques known in the art can be used to quantify the amount of a given protein. These include, but are not limited to immunological techniques such as an ELISA or RIA, or quantitative analytical techniques such as spectroscopy or flame chromatography. Alternatively, the amount of AS160-like-mRNAs could be determined by a hybridization method or a nucleic acid amplification method instead of the amount of protein. Such methods are known to the artisan and include the dot blot hybridization method, the Northern hybridization method or the RT-PCR method.

An alternative detectable signal may be the amount of phosphorylated AKT and/or phosphorylated AS160-like protein, as the degree of phosphorylation of these proteins is indicative for glucose uptake. If the amount or degree of phosphorylation is increased, the glucose uptake of a cell, particularly an insulin-sensitive cell, is increased, too. Methods of determining the amount or degree of phosphorylation are known to the skilled person and include the use of antibodies specific for these phosphorylated proteins as detailed in the Examples.

A further detectable signal may be GLUT4 translocation to the plasma membrane or GLUT4 distribution in a cell, as the degree of translocation of GLUT4 is indicative for glucose uptake. If the amount or GLUT4 in the plasma membrane is increased, the glucose uptake of a cell, particularly an insulin-sensitive cell, is increased, too. Methods of determining GLUT4 translocation to the plasma membrane or GLUT4 distribution in a cell are known to the skilled person and include the use myc-tagged GLUT4 in an In-cell- Western technique or in a ACUMEN technique. These methods may be carried out as detailed in the Examples.

Alternatively, glucose uptake of a cell could be determined, e.g. by using labeled glucose or a labeled glucose derivative. Suitable labels include e.g. detectable tags, radio-active isotopes such as ³H or ¹⁴C or fluorescence markers. Such labeled glucose or a labeled glucose derivatives include without limitation 2-fluoro-2-deoxy-D-glucose, 2-deoxy[¹⁴C] glucose and [¹⁴C]methylglucose. Preferably, radio-labeled 2-deoxyglucose is used. This method may be carried out as detailed in the Examples.

As detailed above, the method of the invention may be used in order to test as substance under high glucose condition, which better reflects the situation in a patient suffering from diabetes. High glucose conditions are those with elevated glucose concentration. The normal / safe level for glucose in the blood of a human is between 3.5 and 7.8 mM. Accordingly, a high glucose condition is a condition with glucose concentration above the normal level. Particularly, the glucose concentration used for the method of the invention may be at least 10 mM, preferably at least 15 mM, more preferably at least 25 mM glucose.

The substance tested with the method of the invention may be any test substance or test compound of any chemical nature. It may be already known as a drug or medicament for a disease other than type 2 diabetes. Alternatively, it may be a known chemical compound not yet known to have a therapeutic effect. In another embodiment the chemical compound may be a novel or so far unknown chemical compound.

In another embodiment of the screening methods of the invention, the test substance is provided in the form of a chemical compound library. Chemical compound libraries include are plurality of chemical compounds and have been assembled from any of multiple sources, including chemically synthesized molecules and natural products, or have been generated by combinatorial chemistry techniques. They are especially suitable for high throughput screening. They may be comprised of chemical compounds of a particular structure or compounds of a particular creature such as a plant. In the context with the present invention the chemical compound library is preferably a library comprising proteins and polypeptides or small molecules.

Advantageously, the method of the present invention is carried out in a robotics system e.g. including robotic plating and a robotic liquid transfer system, e.g. using microfluidics, i.e. channelled structured.

In another embodiment of the present invention, the method is carried out in form of a high-through put screening system. In such a system advantageously the screening method is automated and miniaturized; in particular it uses miniaturized wells and microfluidics controlled by a roboter. High-throughput screening (HTS), is a method for scientific experimentation especially used in drug discovery and relevant to the fields of biology and chemistry.

HTS allows a researcher to effectively conduct millions of biochemical, genetic or pharmacological tests in a short period of time, often through a combination of modern robotics, data processing and control software, liquid handling devices, and sensitive detectors. Through this process one can rapidly identify active compounds which modulate a particular biomolecular pathway; particularly a substance altering the glucose uptake of a cell.

In essence, HTS uses an approach to collect a large amount of experimental data on the effect of a multitude of substances on a particular target in a relatively short time. A screen, in this context, is the larger experiment, with a single goal (usually testing a scientific hypothesis), to which all this data may subsequently be applied.

For HTS, cells comprising AS160-like protein or a nucleic acid coding for the same may be seed in a tissue plate, such as a multi well plate, e.g. a 96-well plate. Then the cell in the plate is contacted with the test substance for a time sufficient to stimulate and generate a suitable detectable signal as defined above. The test substance may be different from well to well across the plate. After incubation time has passed to allow generation of the signal, measurements are taken across all the plate's wells, either manually or by a machine.

Manual measurements may be necessary when the researcher is using microscopy to (for example) seek changes the wells' test compounds, looking for effects that a computer could not easily determine by itself. Otherwise, a specialized automated analysis machine can run a number of experiments on the wells (such as analyzing light of a particular frequency). In this case, the machine outputs the result of each experiment e.g. as a grid of numeric values, with each number mapping to the value obtained from a single well.

Depending upon the results of this first assay, the researcher can perform follow up assays within the same screen by using substances similar to those identified as active (i.e. altering glucose uptake of a cell) into new assay plates, and then re-running the experiment to collect further data, optimize the structure of the chemical compound to improve the effect of the compound on the cell.

Automation is an important element in HTS's usefulness. A specialized robot is often responsible for much of the process over the lifetime of a single assay plate, from creation through final analysis. An HTS robot can usually prepare and analyze many plates simultaneously, further speeding the data-collection process.

A further subject of the invention relates to a test system for the identification of a substance for improving glucose uptake into a cell, the test system comprising
a gene coding for the AKT substrate 160 kDa-like protein (AS160-like protein) or functional variant thereof; and
an inducible promoter providing controllable expression of the gene,
wherein the activation of AS160-like protein effects a detectable signal, and wherein the AS160-like protein
(i) is encoded by the nucleic add having SEQ ID NO: 1,
(ii) is encoded by a nucleic acid encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein,
(iii) is a protein having the amino add sequence according to SEQ ID NO: 3,
(iv) has 99,5% or more sequence homology with SEQ ID NO: 3, or
(v) is a protein having an amino acid sequence according to the sequence NM_014832 but lacking the amino adds 678 to 740 when compared to this AS 160 amino acid sequence,
wherein the test system is in a cell of an insulin-dependent tissue.

It is noted that all features of this test system may be further defined as detailed in connection with the method of the invention.

The test system is particularly useful as it allows for identification of a substance improving (i.e. increasing) glucose uptake of a cell or as a model for studying type 2 diabetes. The combination of AS160-like protein and an inducible promoter providing controllable expression of the gene allows for determining effects in identical cells with and without AS160-like protein. Accordingly, differences in signaling obtained in cells expressing AS160-like protein in comparison to those not expressing AS160-like protein may be assigned to AS160-like protein. As AS160-like protein may be used to identify new potential drugs for type 2 diabetes (as detailed above) or as a key protein in the main insulin-sensitive tissues, these test system may be used to obtain news insights in the pathophysiology and therapy of type 2 diabetes.

The test system of the invention is located in a cell of an insulin-dependent tissue, particularly a genetically engineered cell. The cell any be any of the cells disclosed in the context of the method of the invention. Particularly the gene and/or the promoter may be introduced into the genetically engineered cell.

The test system of the invention comprises an inducible promoter providing controllable expression of the gene. Controllable expression of the gene means that the expression can be induced or repressed upon a chemical or physical stimulus to the test system which can be applied as intended by the investigator or experimenter.

Promoters represent critical elements that can work in concert with other regulatory regions (enhancers, silencers, boundary elements/insulators) to direct the level of transcription of a given gene. An inducible promoter is activated in response to either the presence of a particular compound, i.e. the inducer (chemical stimulus) or to a defined physical condition, e.g. elevated temperature (physical stimulus). Inducible promoters are a very powerful tool in genetic engineering because the expression of genes operably linked to them can be turned on or off as desired.

There are a series of chemically-regulated promoters, including promoters whose transcriptional activity is regulated by the presence or absence of alcohol, tetracycline, steroids, metal and other compounds. Physically-regulated promoters include promoters whose transcriptional activity is regulated by the presence or absence of light and low or high temperatures.

Preferably, chemically-regulated promoters should be derived from organisms distant in evolution to the cell where its action is required. Thus, promoters to be used in mammalian cells are mostly derived from organisms such as yeast, E. coli or Drosophila. Particular examples are alcohol-regulated promoter system (alcohol dehydrogenase I (alcA) gene promoter and the transactivator protein AlcR); tetracycline-regulated promoter system (tetracycline repressor protein (TetR), tetracycline operator sequence (tetO), tetracycline transactivator fusion protein (tTA), which is the fusion of TetR and a herpes simplex virus protein 16 (VP16) activation sequence, the promoter system disclosed in Example 2); steroid-regulated promoter systems (steroid-responsive promoter, e.g. promoters based on the rat glucocorticoid receptor (GR) or promoters based on the human estrogen receptor (ER)); or metal-regulated promoters derived from metallothionein genes from yeast, mouse and human.

However, the inducible promoter is preferably a tetracycline-inducible promoter, more preferably the promoter system as described in Example 2.

A further aspect of the invention relates to the in vitro use of a tests system comprising AS160-like protein for the identification of a substance altering, particularly improving, glucose uptake into a cell as already detailed above in the context of the method or test system of the invention, wherein the AS160-like protein
(i) is encoded by the nucleic acid having SEQ ID NO:1,
(ii) is encoded by a nucleic acid encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein.
(iii) is a protein having the amino acid sequence according to SEQ ID NO: 3,
(iv) has 99,5% or more sequence homology with SEQ ID NO: 3, or
(v) is a protein having an amino acid sequence according to the sequence NM_014832 but lacking the amino acids 678 to 740 when compared to this AS 160 amino acid sequence,
wherein the test system is in a cell of an insulin-dependent tissue. The test system used may be further specified as described in above with respect to the method or test system of the invention.

Also in accordance with the above disclosure AS160-like protein may be used in vitro in a model for type 2 diabetes, wherein the above details with respect to the method or test system of the invention are to be applied accordingly, and wherein the AS160-like protein
(i) is encoded by the nucleic acid having SEQ ID NO:1,
(ii) is encoded by a nucleic acid encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein.
(iii) is a protein having the amino acid sequence according to SEQ ID NO: 3,
(iv) has 99,5% or more sequence homology with SEQ ID NO: 3, or
(v) is a protein having an amino acid sequence according to the sequence NM_014832 but lacking the amino acids 678 to 740 when compared to this AS 160 amino acid sequence,
wherein the test system is in a cell of an insulin-dependent tissue.

A further embodiment of the invention concerns a polypeptide, said polypeptide being
(i) encoded by the nucleic acid having SEQ ID NO:1,
(ii) encoded by a nucleic acid encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein,
(iii) having the amino acid sequence according to SEQ ID NO: 3,
(iv) having 99,5% or more sequence homology with SEQ ID NO: 3 or
(v) having an amino acid sequence according to the sequence NM_014832 but lacking the amino acids, 678 to 740 when compared to this AS 160 amino acid sequence.

In another embodiment, the invention concerns a polynucleotide consisting of the polynucleotide sequence according to SEQ ID NO: 1 or encoding a polypeptide according to SEQ ID NO: 3 or encoding a protein consisting of SEQ ID NO: 3 and short C- and/or N-terminal sequences of at most 30, 25, 20, 15, 10, 9, 8, 7, 6, 5, 4, 3, 2, or 1 amino acids homologous or heterologous to the protein.

Yet another embodiment of the invention concerns an antibody, specifically binding to a polypeptide, said polypeptide being
(i) encoded by the nucleic acid having SEQ ID NO:1,
(ii) encoded by a nucleic acid encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein,
(iii) having the amino acid sequence according to SEQ ID NO: 3,
(iv) having 99,5% or more sequence homology with SEQ ID NO: 3 or
(v) having an amino acid sequence according to the sequence NM_014832 but lacking the amino acids, 678 to 740 when compared to this AS 160 amino acid sequence.

The preparation of suitable antibodies or functional fragments thereof is well known in the art, e.g. by immunizing a mammal, for example a rabbit, with AS 160-like protein or a fragment thereof, where appropriate in the presence of, for example, Freund's adjuvant and/or aluminium hydroxide gels (see, for example, Diamond, B.A. et al. (1981) The New England Journal of Medicine: 1344-1349). The polyclonal antibodies which are formed in the animal as a result of an immunological reaction can subsequently be isolated from the blood using well known methods and, for example, purified by means of column chromatography. Suitable procedures to produce monoclonal antibodies are well known in the art as well (see e.g. Winter, G. & Milstein, C. (1991) Nature, 349, 293-299 and literature for standard methods listed below). In the context of present invention, the term antibody or antibody fragment comprises also recombinant antibodies or antigen-binding parts thereof, e.g. chimaeric, humanized, multifunctional, bispecific, oligospecific or single-stranded antibodies or antibody F(ab) or F(ab)₂ fragments (see, e.g. EP-B1-0 368 684, WO 88/01649, WO 93/06213, WO 98/24884, US 4,816,567 or US 4,816,397).

A specific anti AS 160-like antibody should interact more strongly with AS160-like protein than with the isoform 1 of AS160 under standard laboratory conditions (e.g. in a Western Blot or the like). According to one embodiment described herein, the specific anti AS 160-like antibody interacts more strongly with novel isoform 2 of AS 160, as identified herein, than with one or both of the isoforms 1 or 3 of AS 160 protein under standard laboratory conditions (i.e. a specific AS 160, isoform 2 antibody).

According to another embodiment, the invention concerns a cell heterologously expressing a polypeptide being
(i) encoded by the nucleic add having SEQ ID NO: 1.
(ii) encoded by a nucleic add encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein,
(iii) having the amino acid sequence according to SEQ ID NO: 3,
(iv) having 99,5% or more sequence homology with SEQ ID NO: 3 or
(v) having an amino acid sequence according to the sequence NM_014832 but lacking the amino acids 678 to 740 when compared to this AS 160 amino acid sequence.

According to another embodiment, the invention concerns a cell stably or transiently transfected with a polynucleotide consisting of the polynucleotide sequence according to SEQ ID NO: 1 or encoding a polypeptide according to SEQ ID NO: 3 or encoding a protein consisting of SEQ ID NO: 3 and short C- and/or N-terminal sequences of at most 30, 25, 20, 15, 10, 9, 8, 7, 6, 5, 4, 3, 2, or 1 amino acids homologous or heterologous to the protein.

The cell can be any procaryotic or eucaryotic cell capable of being stably or transiently transfected with a nucleic acid vector and of expressing a heterologous gene. These comprise principal primary cells as well as cells from a cell culture, preferably a eucaryotic cell culture comprising cells derived either from multicellular organisms and tissue (such as HeLa, CHO, COS, SF9 or 3T3 cells) or from single cell organisms such as yeast (e.g. s. pombe or s. cerevisiae), or a procaryotic cell culture, preferably Pichia or E. coli. Cells and samples derived from tissue can be gained by well-known techniques, such as taking of blood, tissue punction or surgical techniques.

Another aspect described herein concerns a siRNA (small inhibitory RNA) capable of negatively interfering with expression and/or activity of any of AS160 like isoforms 2 and/or 3, e.g. specific for or in part complementary to at least a part of the DNA sequence SEQ ID NO:1 or SEQ ID NO.22:
The term "siRNA" refers to small inhibitory RNAs that induce the RNA interference (RNAi) pathway (for the RNAi interference pathway, see e.g. Elbashir et al., Genes and Development (2001) 15: 188-200, Tuschl. et al., (1999), Genes and Development, 13: p. 3191-3197 or Zamore et al, Cell (2000) vol.101, p.25-33). In the context of present invention the term "siRNA" comprises duplexes of two separate strands, as well as single strands that can form hairpin strucures comprising a duplex region, so-called shRNAs - short hairpin RNAs. siRNA molecules can vary in length (in general 15 to 35, 18 to 30 or 20 to 25 nucleotides in length); however, the choice of the appropriate length is well known in the art. Moreover, siRNAs can vary in their degree of complementarity to their target mRNA in the antisense strand. The choice of the appropriate degree of complementarity is also well known in the art. siRNAs may have unpaired overhanging bases on the 5' and/or the 3' end of the sense strand and/or the antisense strand.

Design and preparation of siRNAs for a given cDNA sequence are well known in the art see, for example Elbashir et al. (2001) Nature 411: 494-498 (see especially p.497, right column, for preparation of siRNA and siRNA transfection into cells), and Tuschl et al. (1999) Genes and Development 13: 3191-3197).

Methods for application of siRNA include chemically synthesized or *in vitro* transcribed siRNA, e.g. duplex or shRNA, which are than to be transfected or injected into cells or transgenic animals. SiRNA can also be expressed from expression vectors or PCR products in cells or transgenic animals, wherein the term expression vector refers to any kind of vector system useful for driving expression of siRNAs (either duplex or shRNA) and comprises shuttle vectors as well as viral, such as retro-and lentiviral vectors, as well known in the art.

Another aspect described herein concerns the use of the AS 160-like protein (isoforms 2 and or 3) or the nucleic acid sequence thereof, for generating a siRNA, either duplex or shRNA, able to negatively interfere with expression and/or activity of AS 160-like protein (isoforms 2 and/or 3).

The following figures and examples shall illustrate the present invention.

### FIGURES

Figure 1 shows a comparison of the three different isoforms of AS160. Novel AS160-like protein (isoform 2 as well as novel isoform 3) has been identified on the basis of quantitative RT PCR (Taqman) using primers SEQ ID NO 7, 8, 9 for isoform 2. Full-length AS160 (amplified with primers SEQ ID NO 19, 20 and 21) is depicted in (A), the AS160-like protein, isoform 2, which lacks the exons 11 and 12 in (B). Isoform 3 is shown in (C) (amplified with primers SEQ ID NO 16, 17, 18). Phosphorylation sites Ser-588 and Thr-642 (underlined) and mismatches T202 and T1275 (italic) are shown.
Figure 2 (A and B) shows expression of AS160 isoforms (Taqman). Using specific primers (Primers SEQ ID NO 4, 5, 6 and/or 19, 20, 21) for full-length AS160 , primers SEQ ID NO 7,8, 9 for AS160-like and primers 16, 17, 18 for isoform 3), the expression of the three different isoforms was examined. mRNA levels of AS160 isoforms are normalized against expression of endogenous RPL37a mRNA (amplified with primers SEQ ID NO 10, 11, 12). Data are representative for five different human donors.
Figure 3 shows a schematic presentation of cloning strategy for cloning of isoforms 2 of AS160. The tet-repressor derived from pCDNA3.1 (+)/TR was cloned into Nhel and NotI sites of pIRESpuro2 generating pIRESpuro2/TR.

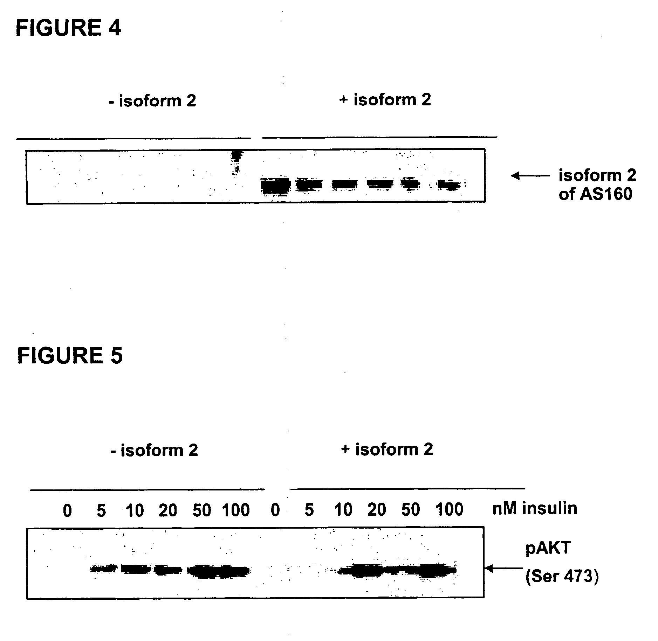

Figure 4 shows that expression of isoforms 2 of AS160 in L6-GLUT4myc cells is tet-inducible. RIPA (radio immuno precipitation assay) extracts were analyzed with SDS-PAGE and western blot analysis. Cells were incubated in the absence of doxycycline (-isoform2) or in the presence of doxycycline (+isoform2) for 48 hours. Expression of AS160-like was detected with a specific AS160 antibody (Upstate, Cat No.: 07-741).
Figure 5 shows insulin stimulated AKT activation in L6-GLUT4myc cells, with or without expression of isoforms 2 of AS160. Phosphorylation of AKT at Ser 473 was confirmed with an anti-pAKT (Ser 473) antibody (Biosource, Cat No.: 44-621G).

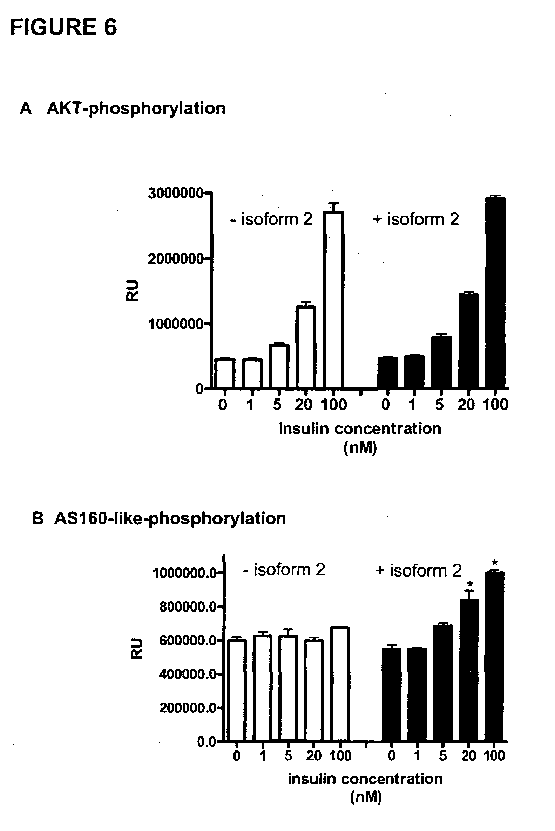

Figure 6 shows phosphorylation of AKT and isoform2 of AS160 (In-cell western). An In-cell western analysis was performed in a 96 well plate. Cells were pre-treated with doxycyclin for 48 hours to induce the expression of isoform2 of AS160 (+ isoform2 of AS160). Insulin was incubated for 20 minutes. The phospho-AKT antibody detects the phosphorylation of AKT at Ser 473 (Biosource, Cat No.: 44-621 G). The phospho-AS160 antibody (Biosource Cat No.: 44-1071 G) is specific for the phosphorylation site at Thr 642. Standard deviations represent 8 reading points. *P values < 0.001 vs. - AS160-like. RU represents relative units.

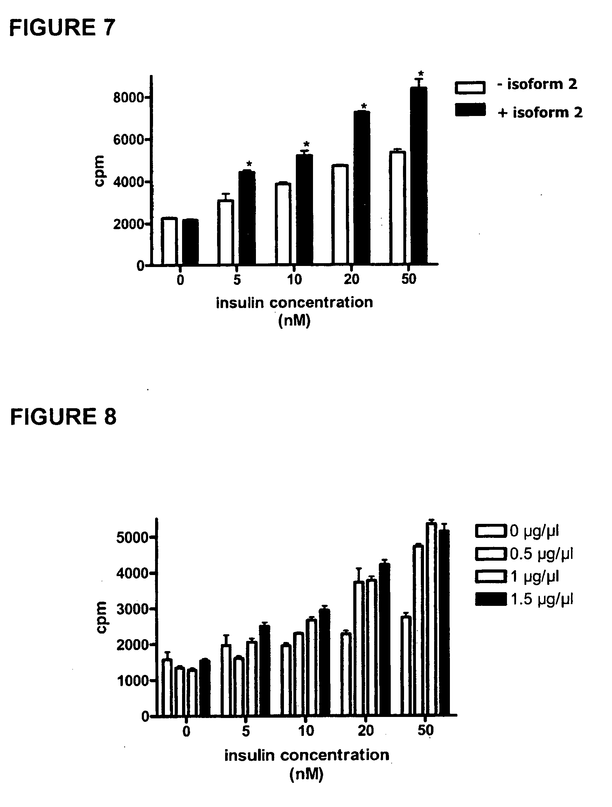

Figure 7 shows the effect of isoform2 of AS160 on insulin stimulated glucose uptake. Uptake of 2-deoxyglucose is measured in response to insulin. Insulin was incubated for 20 minutes. Standard deviations are representative for 8 reading points. *P<0.001 vs. - isoform2 of AS160 -like values.
Figure 8 shows dose-dependency of glucose uptake. Doxycyclin-dependency was investigated with uptake of 2-deoxyglucose of L6-GLUT4myc cells containing isoform2 of AS160. Doxycyclin was incubated for 48 hours. Insulin was incubated for 20 minutes. Standard deviations are representative for 8 reading points.

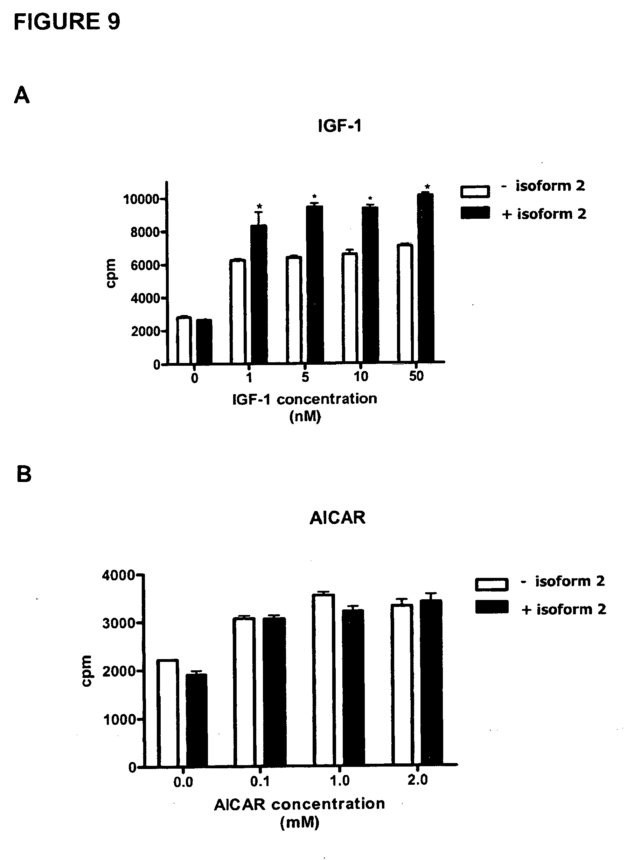

Figure 9 shows the effect of isoform2 of AS160 on IGF-1 and AICAR stimulated glucose uptake. Uptake of 2-deoxyglucose is measured in response to IGF-1 (A) and AICAR (B). IGF-1 was incubated for 20 minutes. AICAR was incubated for 2 hours. Standard deviations are representative for 4 to 8 reading points. *P<0.05 vs.-isoform2 - values.

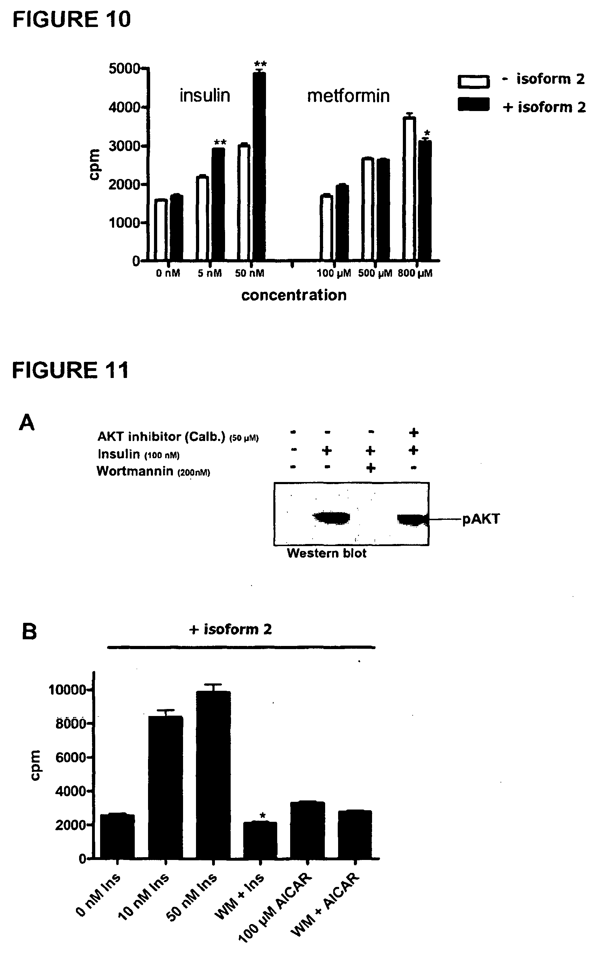

Figure 10 shows the effect of isoform2 of AS160 on metformin-stimulated glucose uptake. Uptake of 2-deoxyglucose is measured in response to insulin (left) and metformin (right). Metformin was incubated during starvation period (3 hours), insulin was incubated for 20 minutes. Standard deviations are representative for 4 reading points. *P<0.05 vs. -AS160-like values, **P<0.001 vs. -AS160-like values.
Figure 11 shows the effect of AKT inhibitors. 10 µg RIPA extract were separated by SDS-PAGE and analyzed by western blotting with a pAKT (Ser 473) specific antibody (Biosource, Cat No.: 44-621G) (A). Cells were pre-incubated with either wortmannin (200nM; Upstate, Cat No.: 12-338) or the AKT inhibitor Calb. (50 µM, Calbiochem, Cat No.: 124005) for 1 hour and subsequently stimulated with insulin (10 nM) for 20 minutes. Uptake of 2-deoxyglucose (B) was measured in response to insulin and AICAR alone and in combination of wortmannin plus insulin or AICAR (Biomol). Insulin was incubated 20 minutes, AICAR was incubated for 2 hours. *P<0.001 vs. -10 nmol insulin.

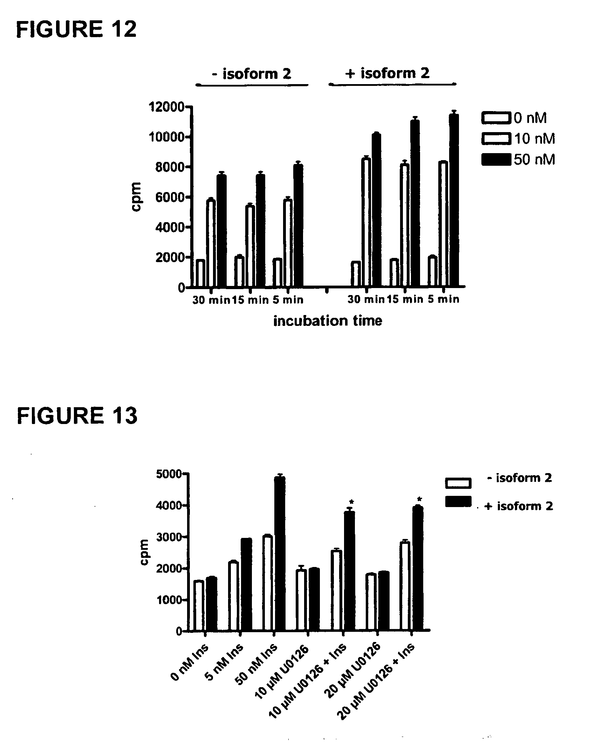

Figure 12 shows the time-dependency of glucose uptake. The uptake of 2-deoxyglucose was measured after 5, 15, 30 minutes. Cells were pre-treated with doxycyclin in order to induce expression of AS160-like protein or left untreated. Standard deviations were obtained from 8 independent values.
Figure 13 shows the effect of MEKK/ERK inhibitor on glucose uptake. Expression of isoform2 of AS160 was induced with doxycyclin for 48 hours (+AS160-like). Cells were starved for 4 hours. The MEKK/ERK inhibitor U0126 (10 µM, 20µM, Upstate Cat No.: 19-147) was incubated in starve medium. Subsequently, cells were stimulated for 20 minutes with the indicated insulin concentrations. *P<0.05 vs. -AS160-like.

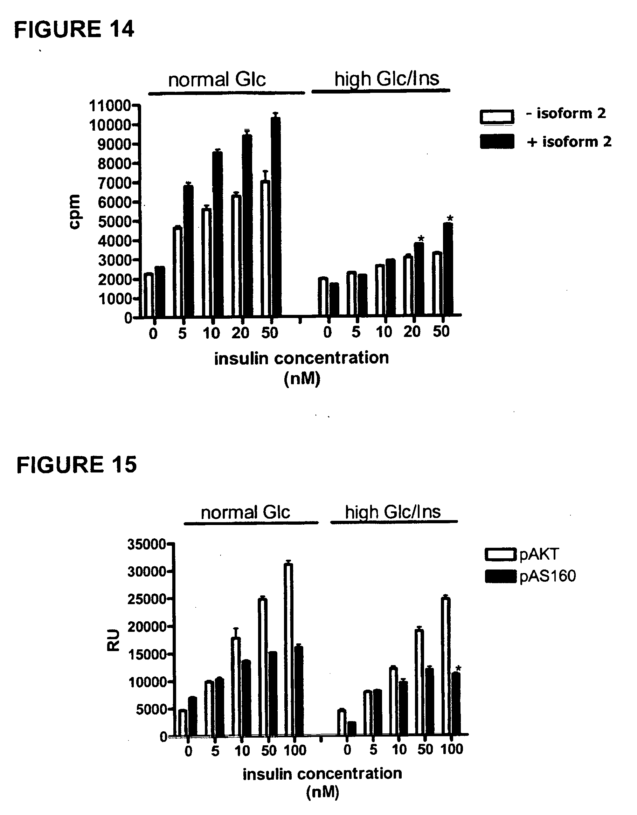

Figure 14 shows the induction of insulin-resistance (glucose uptake). To induce insulin-resistance cells were incubated in the presence of high glucose and insulin overnight (right), or incubated under normal conditions (left). Induction of isoform2 - expression was performed by incubation of doxycyclin for 48 hours. Insulin was incubated for 20 minutes. Standard deviations represent 8 reading points. *P< 0.05 vs. - isoform2 of AS160.
Figure 15 shows induction of insulin-resistance (In-Cell western). To induce insulin-resistance cells were incubated in the presence of high glucose plus insulin overnight or incubated under normal conditions. Induction of isoform2-expression was performed by incubation of doxycyclin for 48 hours. Activation of AKT was determined with an antibody recognizing Ser 473 (Biosource Cat No.: 44-621 G). The phospho-AS160 antibody is directed against the Thr 642 site. *P<0.05 vs. 100 nM insulin under normal conditions.

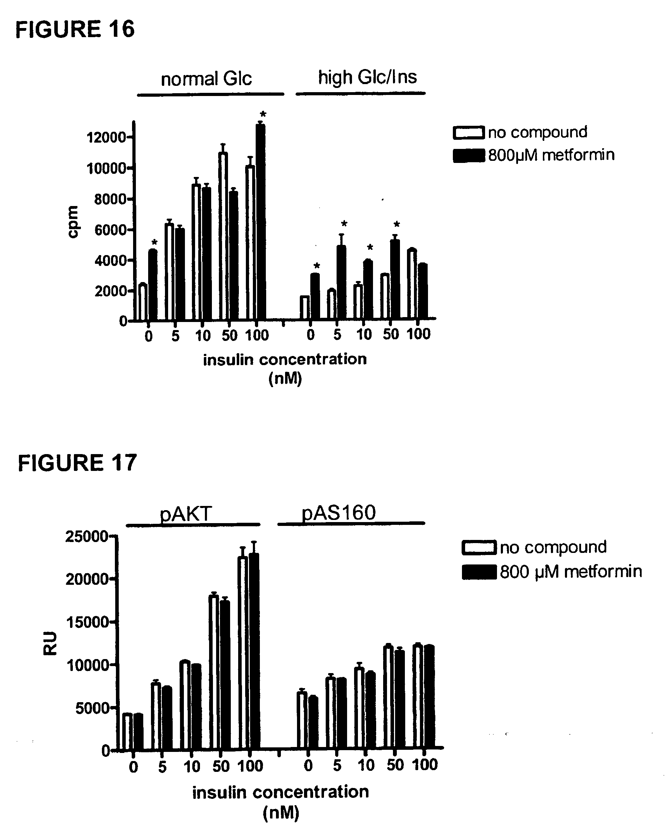

Figure 16 shows the effect of metformin on glucose uptake. Expression of isoform2 of AS160 in all wells was induced with doxycyclin for 48 hours. Insulin-resistance was induced overnight. Metformin (800 µM) was incubated overnight. Cells were starved for 4 hours and subsequently stimulated with different insulin concentrations under indicated conditions. *P<0.05 vs. appropriate values without compound.
Figure 17 shows the effect of metformin on phosphorylation of AKT and AS160, isoform 1 (In-cell Western). Expression of isoform2 of AS160 in all wells was induced with doxycyclin for 48 hours. Insulin-resistance was induced overnight. Metformin (800 µM) was incubated overnight. Cells were starved for 4 hours and subsequently stimulated with different insulins with the indicated conditions. RU represents relative units.

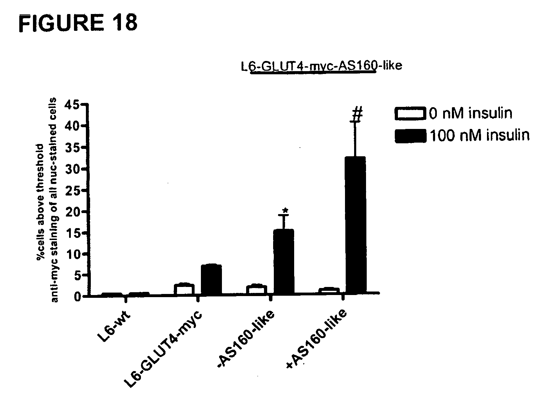

Figure 18 shows an analysis of GLUT4 translocation (ACUMEN). Cells were incubated with doxycyclin to induce expression of AS160-like protein or left untreated. Insulin was incubated for 20 minutes. Standard deviations were obtained from 8 single values. *P< 0.05 vs. L6-GLUT4-myc + 100 nM insulin. #P< 0.05 vs. - isoform2 of AS160 + 100 nM insulin.

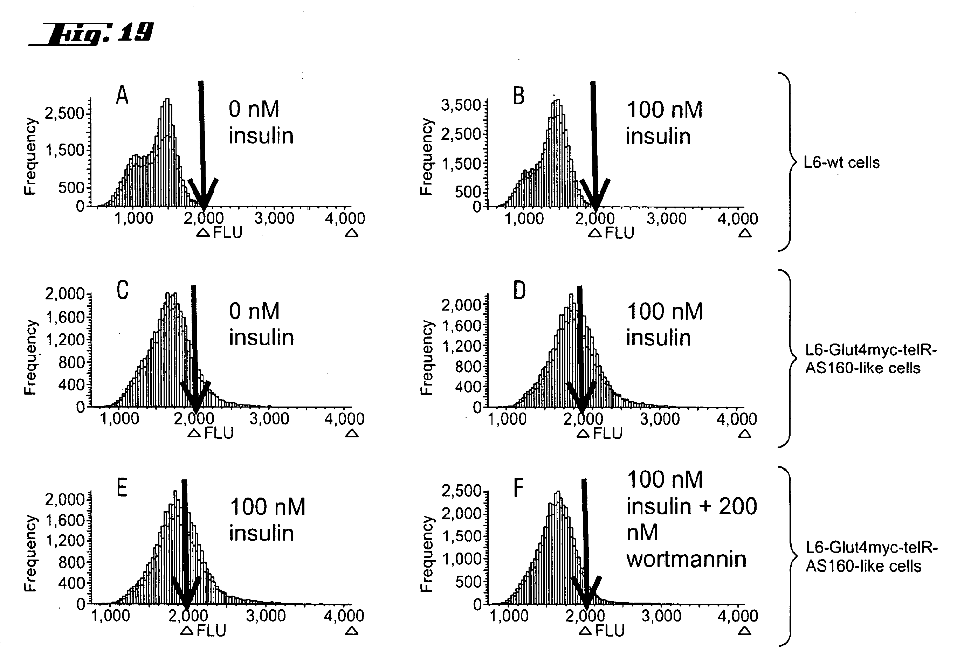

Figure 19 shows a graphic presentation of GLUT4 distribution (ACUMEN). Histograms were obtained on the basis of laser-scanning fluorescence cytometry after different conditions. The curves represent total cell counts (DNA marker). Cells left of the arrow shown in lighter grey are negative for myc-GLUT4 staining. Cells right of the arrow shown in lighter grey represent the cell population that display translocated myc-tagged GLUT4 at the plasma membrane.

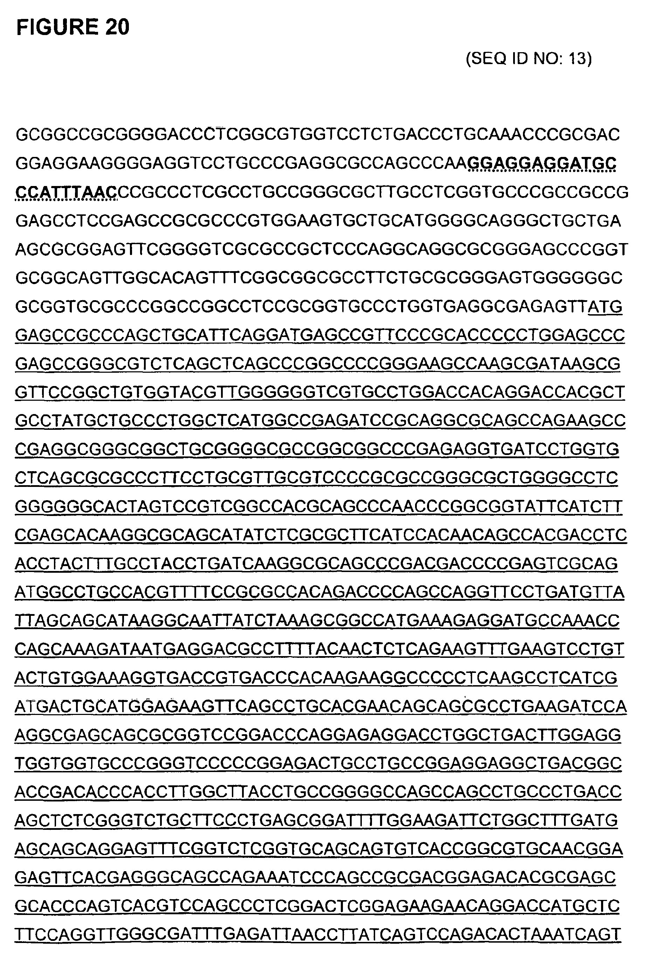

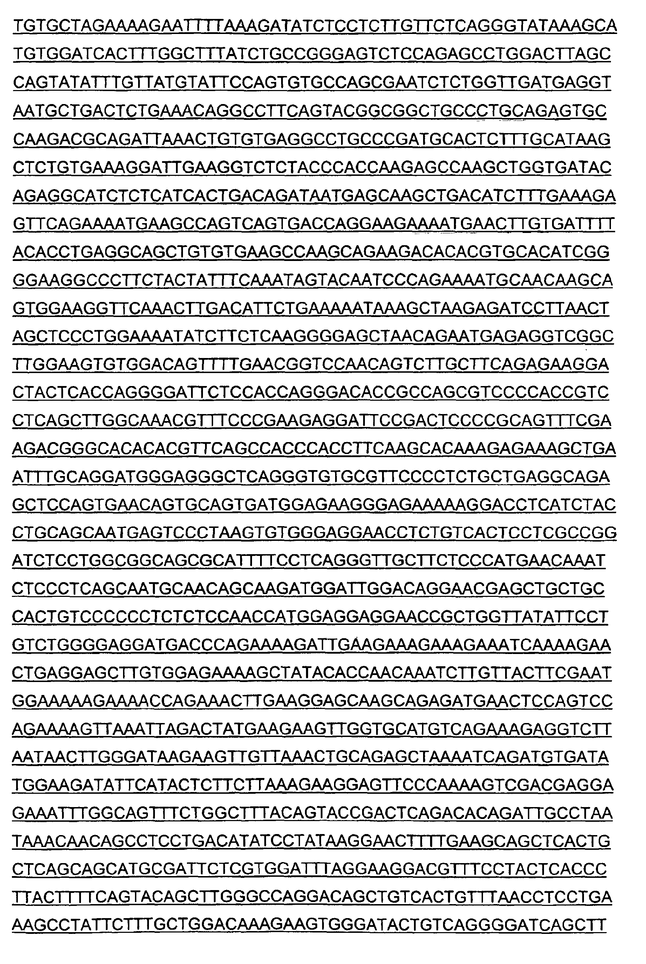

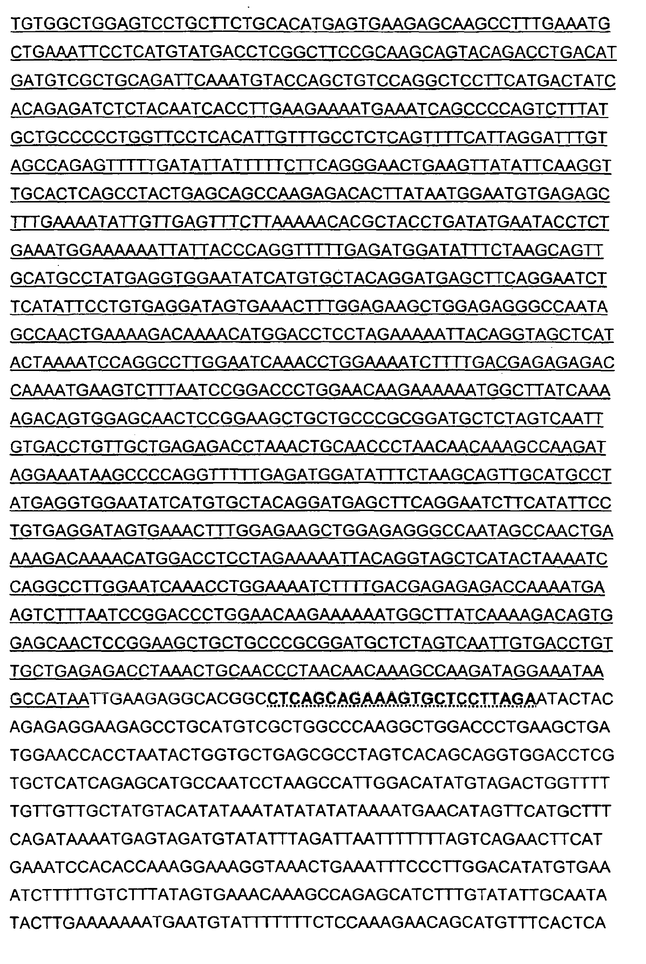

Figure 20 shows the location of the forward and reverse primers for PCR-cloning of isoform2 of AS160 from human testis cDNA on the DNA sequences upstream and downstream of the isoform2 - coding Sequence (SEQ ID NO: 13). Primers are typed bold and underlined by dotted lines; the AS160-like coding sequence is underlined by a solid line.

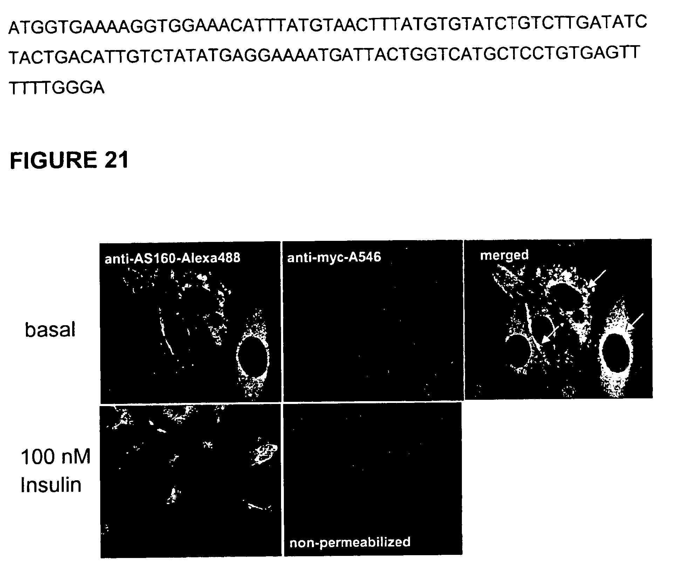

Figure 21 shows an immunofluorescence based analysis of the intracellular localization of myc-GLUT4 and isoform2 of AS160 under basal and insulin stimulated conditions. Rat myoblast cells were incubated with insulin (100 nM) for 20 minutes prior to treatment for immunofluorescence-microscopy.

### EXAMPLES

### Example 1: Expression of AS160 isoforms in different tissues

Bioinformatical analysis of ESTs indicated the presence of three isoforms of AS160 (Figure 1A ,B, C). Figure 1 presents a schematic overview of AS160 and the new isoforms identified. The expression of these different AS160-isoforms was investigated with quantitative RT-PCR in different tissues. Commercially available RNAs of five different human donors were reverse transcribed and examined using Taqman PCR with specific primer pairs (for full-length AS160 SEQ ID NO: 4,5 and 6, (or alternatively 19,20 and 21) for AS160-like SEQ ID NO: 7,8 and 9, for isoform 3 SEQ NO: 16, 17 and 18).

For this, aliquots of total cellular RNA were subjected to first-strand DNA synthesis. Reverse-transcribed cDNA was used as a template for amplification. A common probe was used to determine the overall AS160 expression in insulin-sensitive tissue (adipose, muscle, liver, heart, brain). Endogenous mRNA expression of the ribosomal gene RPL37a (Homo sapiens ribosomal protein L37a, mRNA, cDNA clone MGC:26772) was used to normalize mRNA levels (SEQ ID NO: 10, 11 and 12). Based on specific primer pairs the expression of distinct isoforms could be distinguished. Relative mRNA expression methods were calculated with the deltadelta CT method (Yuan et al., 2006).

The following primers and probes were used:

| Specificity | Sequence | SEQ ID NO: |
|---|---|---|
| Isoform 1 (full-length AS160) | For 5'-ATTCAGGTAGACTGTCCCCACAGTAT | 19 |
| | Rev 5'-CCTTCTCCATCACTTGATTCTGAAG | 20 |
| | Probe: FAM-MGBNFQ | 21 |
| | 5'-ATGAAATCAGACAAGACACTG | |
| Isoform 2 (AS 160-like) | For 5'- GCGTTCCCCTCTGCTGAG | 7 |
| | Rev 5'-ACTCATTGCTGCAGGTAGATGAG | 8 |
| | Probe: FAM-TAMRA | 9 |
| | 5'-TTCTCCATCACTGCACTGTTCACTGGAGCT | |
| RPL37a | For 5'-ACAGCGGAAGTGGTATTGTACGT | 10 |
| | Rev 5'-GGCACTGTGGTTCCTGCAT | 11 |
| | Probe: VIC-TAMRA | 12 |
| | 5'-CAGGCACCGCCAGCCACTGTCT | |
| PCR cloning of Isoform2 | For 5'- GGAGGAGGATGCCCATTTAAC- 3 (SEQ ID NO: 14) | 14 |
| cDNA | | |
| | Rev 5'-TCTAAGGAGCACTTTCTGCTGAG-3' | 15 |
| Isoform 3 | For 5'- AGCTTTTACCAGAATTCAGGTAGACTGT | 16 |
| | Rev 5'- TGCTGCAGGTAGATGAGGTCCT | 17 |
| | Probe: FAM-MGBNFQ | 18 |
| | 5'- CTTCTCCATCACTGATTTCATT | |
| Isoform1 (full-length AS160) | For 5'- CATACTCTTCTTAAAGAAGGAGTTCCCA | 4 |
| | Rev 5'- CTGTGTCTGAGTCGGTACTGTAAAGC | 5 |
| | Probe: FAM-TAMRA | 6 |
| | 5'- CAGAAACTGCCAAATTTCTCCTCGTCGACT | |

The results of this RT-PCR are shown in Figure 2. Isoform 1 (NM_014832_v1; EMBL) represents full-length AS160. Full-length AS160 as well as isoform 3 are mainly expressed in heart and skeletal muscle (Figure 2A and B). Isoform 2,, which represents the AS160-like, is mostly expressed in adrenal and thyroid glands, but also in lung kidney and brain (Figure B). Expression is also detected in adipose tissue and in the liver (Figure 2 A). In comparison to full-length AS160 and isoform 3, AS160-like is only slightly expressed in skeletal muscle. The data might indicate a specific function of distinct isoforms in different tissues. In the following experiments the inventors focused on the examination of isoform 2 (AS160-like), since it is novel and shows highest overall expression in most tissues.

Example 2: Cloning of novel AS160-like protein expression construct and establishment of a tetracycline-inducible AS160-like protein expression system AS160-like insert (SEQ ID NO: 1) was amplified from human testis cDNA (Clontech, Cat# 7117-1; Lot#2100009) using primers according to figure 21 (SEQ IDs 14 and 15); the obtained PCR fragment (see figure 21, the insert is underlined) was used as a template for generating a fragment corresponding to the coding sequence having gateway sequences for cloning. The insert was cloned into pDONR221 (Invitrogen) and subsequently into the expression vector pCDNA5-TO (Invitrogen) by means of standard methods. Sequencing analysis revealed that in comparison to full-length AS160 (NM_014832; EMBL) exons 11 and 12 are missing in AS160-like (Figure 1). Two mismatches were identified at positions nt606 (silent) and nt3827 (Ala → Val). In addition, this clone contains a 3 bp deletion (nt 2594-2596), that was also found in human placenta cDNA but not in human brain cDNA. For the expression clone of the isoform lacking exons 11 and 12 the 3bp deletion was repaired to resemble more closely the full length sequence of NM_014832 (EMBL).

L6 myoblasts (rat skeletal muscle cells) stably expressing glucose transporter 4 (GLUT4) with an exofacially directed myc-tag (GLUT4myc) (L6-GLUT4myc, described in Wang et al. 1998) were subsequently used for tetracycline (tet) -inducible expression of AS160-like protein. For this purpose the T-REx system from Invitrogen was used. The regulatory plasmid in this system controls the constitutive expression of the tet-repressor (tet-R) under the control of a CMV (cytomegalovirus) promoter. In the absence of tetracyline (or doxycycline) the repressor binds to specific tetracycline-operator sequences (TetO2) and thereby represses expression. Addition of tetracycline (or doxycycline) induces expression of the protein of interest. To allow a stable integration of the tet-system in L6-GLUT4myc cells the tet-repressor was isolated from pCDNA3.1 (+)/TR (Invitrogen) and cloned into the Nhel and Notl sites of pIRESpuro2 as shown in figure 3. The sequence of pIRES-puro2/TetR is given in the following as SEQ ID NO: 2.
Sequence: pIRES-puro2/TetR (SEQ ID NO: 2)

Subsequently pIRESpuro2/TR was transfected into L6-GLUT4myc cells. Clones stably expressing the regulatory plasmid were selected with 0.5 µg/ml puromycin (InvivoGen). L6-GLUT4myc cells containing AS160-like were grown in MEMα (PAN) supplemented with 10% fetal calf serum (FCS) (PAA, tet-free), 2 µg/ml blasticidin (Calbiochem), 0.5 µg/ml puromycin (InvivoGen), 200 µg/ml hygromycin (Invitrogen). Functionality of the tet-repressor was controlled with a tetracycline-inducible GFP expression plasmid (pCDNA5/TO-GFP). In the absence of tetracycline (or doxycyclin) only a small number of cells express GFP. Addition of doxycyclin increases the number of GFP expressing cells about 10 fold (data not shown).

To obtain a tet-inducible expression of the isoform2 of AS160, the pCDNA5 vector from Invitrogen containing the gene of isoforms 2 of AS160 was used. Selection of stable clones was performed with hygromycin (200 µg/ml, Invitrogen). Expression of isoform2 of AS160 was examined via western blot analysis with an AS160-specific antibody recognizing full-length AS160 and isoform2 of AS160. Expression of isoform2 of AS160 was induced with 1 µg/ml doxycyclin (Sigma). Functionality of the tet-repressor was investigated with addition of doxycyclin (1 µg/ml, Sigma) and subsequent western blot analysis.

For all examples L6-GLUT4myc cells containing isoform2 of AS160 were grown in MEMα (PAN) supplemented with 10% fetal calf serum (FCS) (PAA, tet-free), 2µg/ml blasticidin (Calbiochem), 0.5µg/ml puromycin (InvivoGen), 200µg/ml hygromycin (Invitrogen). Expression of isoform2 of AS160 was induced with 1µg/ml doxycyclin (Sigma). L6-wildtype (wt) (ATCC: CRL-1458) cells were grown in MEMα + GlutaMax (Gibco) supplemented with 10% FCS (PAA, tet-free) and 1% penicillin/ streptomycin (PAA). L6-GLUT4myc cells were grown in MEMα + GlutaMax (Gibco) supplemented with 10% FCS (PAA, tet-free), 1% penicillin/streptomycin (PAA) and 2 µg/ml blasticidin (Calbiochem). All cells were grown at 37°C and 5% CO₂. L6-GLUT4myc cells containing isoform2 of AS160 were incubated in starve medium (MEMα) 3-4 hours prior to each experiment.

For Western blot analysis proteins were separated on SDS-PAGE gels (4-12% resolving gel, Invitrogen), transferred to PVDF membranes (Roche) and blocked with Roti-Block® (Roth) for 1 hour. Membranes were incubated with primary antibodies overnight. The anti-AS160 antibody was from Upstate. Membranes were washed in TBST and incubated with the appropriate secondary horseradish peroxidase conjugated antibody (Santa Cruz). Immunoreactive bands were visualized with LumiLight (Roche) and detected with Lumi-Imager (Böhringer Ingelheim).

Analysis of the functionality of the tet-repressor revealed that isoform2 of AS160 is expressed only in the presence of doxycycline (Figure 4).

In order to examine tetracycline- and insulin-dependent expression of isoform2 of AS160 protein in L6-GLUT4myc cells containing AS160-like, a western blot analysis was performed. The expression of AS160-like protein was induced with 1 µg/ml doxycyclin for 48 hours as described above. Subsequently, the cells were stimulated with insulin (5 nM to 50 nM; Sanofi-Aventis) for 20 minutes. Cell extracts were prepared, separated via SDS-PAGE and transferred to a PVDF membrane as described above.

Figure 4 shows a representative western blot of doxycyclin-inducible expression of AS160-like protein in L6-GLUT4myc cells containing the AS160-like transcript. Incubation with insulin induced the phosphorylation of AKT (Ser 473) (Figure 5). Expression of the isoform2 of AS 160 had no effect on the activation of AKT.

### Example 3: Phosphorylation of AKT and AS160

To study phosphorylation of AKT and AS160 the cells described in Example 2 were used. AS160 is activated by phosphorylation on critical motifs (RXRXXSIT). Known phospho-sites in AS160 are Ser 570, Ser 588, Thr 642 and Thr 751 (Sano et al., 2003). One of the kinases responsible for the activation of AS160 is AKT (Kane et al., 2002). In order to determine the activation status of the isoform2 of AS160 the In-cell western blot technique was used. This method allows the detection of specific proteins directly in 96 well plates without preparation of cell extracts. The specific antibody used in this assay recognizes the phosphorylated Thr 642 phosphorylation site of AS160 and AS160-like protein.

For this, cells were seeded into 96-well plates (black, Nunc) and grown for 48 hours. Cells were starved for 3-4 hours with MEMα (PAN) containing 2% horse serum (Cambrex). After removal of medium cells were fixed in 3.7% freshly prepared para-formaldehyde (Sigma) for 20 minutes. Cells were permeabilized with PBS + 0.1% Triton-X-100. Blocking was performed with Odyssey blocking buffer (Licor) overnight at 4°C. Primary antibodies were incubated for 2 hours at room temperature. The anti-phosphoAKT (Ser 473, Cat No.: 44-621 G) and the anti-phosphoAS160 (Thr 642, Cat No.: 44-1071 G) were from Biosource. After incubation of the primary antibody, cells were washed with PBS + 0.1 % Tween20. The secondary anti-rabbit-IgG-800-CW antibody (Rockland Cat No.: 611-131-122) was incubated for 1 hour. For detection of DNA TO-PRO3 dye (Molecular Probes, Cat No.: T3605) was used. Fluorescence signals (Figure 6) are presented as relative units (RU).

As a control the dose-dependent phosphorylation/activation of AKT on Ser 473 was determined in the same experimental setting. Insulin induces AKT phosphorylation and phosphorylation of full-length or isoform2 of AS 160 in a dose-dependent manner (Figure 6A and B). No phosphorylation of AS160 or isoform2 of AS160 protein was observed in cells without doxycyclin-induced expression of AS160-like protein.

### Example 4: Effect of isoform2 of AS160 protein on glucose uptake

To examine glucose uptake of the cells, the respective cells were plated in 96 well Cytostar-T scintillating microplates (Amersham). After 48 hours cells were serum-starved (3-4 hours) and treated with inhibitors as indicated. Uptake of 2-deoxyglucose (0.01 MBq per well, Amersham) was performed as already described (Voss et al., 2005). Nonspecific uptake was determined in the presence of 40 µM cytochalasin B (Calbiochem). This value was subtracted from all other values. Measurement occurred in a Wallac Microbeta counter (Perkin Elmer). Uptake of 2-deoxyglucose is presented as counts per million (cpm):

The uptake of 2-deoxyglucose in L6-GLUT4myc cells expressing isoform 2 of AS160 was examined in response to insulin (Figure 7). The data show that expression of isoform2 of AS160 increases the uptake of glucose up to 4 fold after stimulation with insulin (concentration of 50 nM insulin). Without expression of isoform 2 of AS160 insulin induces glucose uptake up to a maximum of 2 fold.

The increase of glucose uptake in these cells was induced by doxycyclin in a dose-dependent manner (Figure 8) which correlated with isoform2 of AS160 protein levels. From the literature it is already known that IGF-1 (insulin like growth factor-1, R&D Systems, Cat No.: 291-G1) and the AMPK (5'-AMP-activated protein kinase) activator AICAR (5-Aminoimidazole-4-carboxaide 1-beta-D-ribofuranoside, Biomol Cat No.: EI-330) also stimulate glucose uptake in skeletal muscle cells (Ciaraldi et al., 2002). Therefore, we examined the effects of the expression of isoform 2 of AS160 on IGF-1 and AICAR stimulated glucose uptake (Figure 9). In the absence of isoform 2 of AS160, compared to insulin the uptake of glucose in cells stimulated with IGF-1 is higher (3 fold) than after stimulation with insulin (2 fold) (Figure 9 A).

The uptake of glucose in cells stimulated with AICAR is lower (1.5 fold) than the uptake of cells stimulated with insulin (Figure 9 B). Expression of isoform 2 of AS160 protein further increased the uptake of glucose after stimulation with IGF-1 (up to 5 fold), whereas no additional effect of expression of isoform 2 of AS160 was observed after stimulation with AICAR. These data indicate that stimulation of glucose uptake can occur via AMPK in a manner independent of isoform 2 of AS160 and via AKT in an isoform2-dependent manner. Thereby the cell system can be used to differentiate between isoform2-dependent and -independent effects.

### Example 5: Effect of Metformin on glucose uptake

The glucose-lowering effects of metformin (dimethylbiguanide) in type 2 diabetes are already well documented (Karlsson et al., 2005a); however, its exact mechanism of action is uncertain despite its known therapeutic benefits. We examined the effect of isoform 2 of AS160 protein on glucose uptake after stimulation with different concentrations of metformin, wherein the test was carried out as described above (Figure 10). In comparison to insulin, the expression of isoform2 of AS160 protein has no increasing effect on glucose uptake. This effect seems to be comparable with the effect observed after stimulation with AICAR. These data suggest that metformin-induced effects are also independent from isoform 2 of AS160 protein and might be mediated by AMPK..

### Example 6: Effect of AKT inhibitors

To obtain a more detailed analysis of the signaling cascade leading to the activation of isoform 2 of AS160, two different inhibitors were tested. Wortmannin (Upstate Cat No.: 12-338) is an already well established compound known to inhibit at the level of the P13-kinase (Pl3K) and subsequently leading to a reduced phosphorylation of AKT, which signals downstream of PI3-kinase (Okada et al., 1994). The second compound used is 1 L-6-hydroxymethyl-chiro-inositol 2-(R)-2-O-methyl-3-O-octadecylcarb6nate (abbreviated with AKT inhibitor Calb.; Calbiochem Cat No.: 124005). This compound is described as a selective inhibitor of AKT only weakly interfering with Pl3K (Hu et al., 2000).

As expected, Western blot analysis revealed that wortmannin completely abrogated the phosphorylation of AKT (Ser 473) (Figure 11 A). The AKT inhibitor Calb. had no effect on the phosphorylation of AKT (Ser 473) (Figure 11 A). To examine the efficacy of the AKT inhibitor Calb., an analysis of signaling molecules downstream of AKT is required, because the phosphorylation of AKT might not directly correlate with the activity of AKT. Phosphorylation of AKT on Ser 473 was detected with a phospho-specific antibody from Biosource (Cat No.: 44-621 G).

Wortmannin was also able to completely abrogate the uptake of glucose of L6-GLUT4myc cells expressing isoform 2 of AS160, whereas glucose uptake of AICAR stimulated cells remained nearly unchanged (Figure 11 B). So far, the process of glucose uptake appears to be mediated by the Pl3K-AKT signaling pathway.

### Example 7: Time-dependency of glucose uptake of L6-GLUT4-myc cells expressing isoform2 of AS160

The contribution of isoform2 of AS160 responsible for improved glucose uptake might be the acceleration of the translocation of GLUT4 to the plasma membrane, a higher overall amount of GLUT4 protein at the membrane or an impaired endocytosis. In order to elucidate this mechanism a time-dependent analysis of glucose uptake in cells expressing isoform2 of AS 160 with or without induction of isoform2 of AS 160 was performed. Glucose uptake at 3 different time-points was investigated (5, 15, 30 minutes). Cells were stimulated with 2 different insulin concentrations (10 nM, 50 nM). Figure 12 shows that the maximum of glucose uptake is already observed after 5 minutes stimulation with 50 nM insulin. A difference between L6-GLUT4myc cells expressing isoform2 of AS 160 and cells without isoform2 of AS 160 expression could not be detected. These data suggest that isoform 2 of AS 160 does not accelerate glucose uptake within the examined time points.

Another potential reason for the enhanced uptake of glucose in the presence of isoform2 of AS 160 might be an increased amount of GLUT4 at the plasma membrane. Translocation of GLUT4 is a key event in the induction of glucose uptake.

### Example 8: Effect of MEKK/ERK inhibitor U0126

To study the role of mitogen-activated kinases (MAPK) in the negative regulation of insulin-signaling as well as a contribution to insulin-resistance in skeletal muscle cells, we examined the effect of a commonly used MEKK/ERK inhibitor U0126 (Upstate Cat No.: 19-147 ; DeSilva et al., 1998) on glucose-uptake. Incubation of cells with the MEKK/ERK inhibitor U0126 (10 µM, 20 µM) revealed a significantly improved uptake of glucose after additional stimulation with 5 nM insulin. This effect mainly occurred in cells expressing isoform2 of AS160 (Figure 13).

This finding indicates that the MEKK/ERK kinases also negatively influence insulin signaling and glucose uptake in an isoform2-dependent manner in the cell model used. Similar data were recently published by the group of J. Zierath (Bouzakri and Zierath, 2007) for a TNF-α induced insulin-resistance cell model, which speculate that silencing of especially MAPK4 could be a novel approach to restore appropriate insulin signaling in skeletal muscle cells.

### Example 9: Establishment of an insulin-resistance cell model

As in type II diabetes peripheral insulin-resistance becomes immanent, we aimed to establish a cell-based insulin-resistance model that allows studying the molecular basis of insulin-resistance and in parallel allows developing strategies to restore insulin-sensitivity. Cell-culture based models of insulin-resistance are already well established for adipocytes (Nelson et al., 2002; Greene et al., 2001) and L6 myotubes (Walgren et al., 2003).

In this model, cells were grown under high glucose/insulin conditions (25 mM glucose + 10 nM insulin) overnight to induce insulin resistance (Walgren et al., 2003). Compared to cells grown under normal glucose conditions (Figure 14, left), glucose uptake of cells treated with high glucose and insulin, is markedly reduced (Figure 14, right). Expression of isoform 2 of AS160 slightly improves glucose uptake but is not able to restore insulin sensitivity.

In-cell western blot analysis revealed that also under high glucose plus insulin conditions AKT (Ser473, Biosource Cat No.: 44-621 G) and AS160 (Thr 642, Biosource Cat No.: 44-1071G) are still phosphorylated in a dose-dependent manner (Figure 15). However the phosphorylation signal is decreased to 72% compared to untreated conditions (normal Glc).

In addition, the effect of metformin under high glucose conditions was examined. For this purpose metformin (800 µM) was incubated overnight under normal conditions and in parallel under high glucose plus insulin conditions (25 mM glucose + 10 nM insulin). Isoform2-expression was induced in all wells. Figure 16 shows that metformin significantly enhanced basal glucose uptake under normal conditions. Additional stimulation with different insulin concentrations with exception of 100 nM insulin did not further improve glucose uptake under normal conditions, indicating that metformin has no sensitizing effect.

Under high glucose plus insulin conditions metformin significantly increased basal glucose uptake as well as glucose uptake after stimulation with 5, 10 and 50 nM insulin (Figure 16, right). However, the maximum of glucose uptake under insulin-resistant conditions does not exceed the basal levels after metformin stimulation, demonstrating again that metformin action in muscle is independent from insulin-stimulated glucose disposal.

Parallel examination of the phosphorylation status of AKT and AS160 under normal und insulin-resistant conditions revealed that treatment of cells with metformin has no effect on activation of AKT or AS160 (Figure 17). These data indicate that metformin mediated effects do not depend on AKT or AS160 activation.

### Example 10: Examination of GLUT4 translocation

Although the cell-based glucose uptake is very reproducible and allows for a highly sensitive screening of compounds, it might still be improved for a high throughput screening (HTS). One of the rate-limiting steps in this context is the usage of radioactively labeled glucose in the uptake experiments. To provide an improved basis for this assay in HTS screening, we examined the correlation between increased glucose uptake and translocation of GLUT4 to the plasma membrane.

For this purpose, a technique suitable for screening of compounds was applied. Laser-scanning fluorescence microplate cytometry (ACUMEN technique, LIT) allows the distinct multiparametric analysis of single fluorescent cells in microplates (Bowen and Wylie, 2006). For laser-scanning fluorescence microplate cytometer (Acumen) cells were plated in 96 well plates (Biocat, black). Serum-starved cells were treated as indicated. Briefly, cells were fixed in 3.75 % para-formaldehyde (Sigma) for 20 minutes. Subsequently, quenching occurred with 100 nM NH₄Cl. Cells were blocked with an adequate blocking solutions for a minimum of 1 hour. Primary antibody (monoclonal anti-myc 9E10, Santa Cruz, sc-40) was incubated for 1 hour. The secondary goat-anti mouse IgG (Alexa Fluor 488, Molecular Probes) was incubated in the presence of Sytox-orange for 1 hour (Bowen and Wylie, 2006).

Based on this highly sensitive procedure it is possible to screen various cell numbers for plasma-membrane based GLUT4-myc-display. Cells were counter-stained with a DNA dye (SytoxOrange) to confirm equal cell numbers.

As an additional control, the parental cell lines, L6-wt and L6-GLUT4-myc were included in this experiment. The ACUMEN experiment revealed a significantly increased translocation of GLUT4 to the plasma membrane (-15 %) (Figure 18). As expected GLUT4 translocation is already slightly induced after stimulation with 100 nM insulin alone. Translocation is also increased in cells that contain the AS160-like expression cassette, but were not induced with doxycyclin. This phenomenon probably results from a leaky promoter.

A graphic presentation of GLUT4 translocation obtained with the laser-scanning fluorescence microplate cytometer (ACUMEN) is shown in figure 19. In these histograms the curves represents the total number of detected cells stained with a DNA marker (SytoxOrange). Cells left of the arrow shown in lighter grey do not display plasma-membrane bound GLUT4. Cells right of the arrow shown in lighter grey represent the population of cells that display the myc-tagged GLUT4 at the plasma-membrane.

### Example 11: Localization of GLUT4 and isoform2 of AS160

Immunofluorescence based analysis was applied to detect the localization of GLUT4-myc and AS160-like in the rat myoblast cell line L6-GLUT4-myc-tetR-AS160-like.

Cells were grown on sterile cover slides in 12 well plates. Expression of isoform2 of AS 160 was induced with doxycyclin treatment for 48 hours. Serum-starved cells were treated with 100 nM insulin for 20 minutes or were left untreated. After stimulation, cells were fixed in 3.75 % para-formaldehyde (Sigma) for 20 minutes. Subsequently, cells were permeabilised with 0.1% TritonX100 in PBS (Icon Biomedicals) or left unpermeabilised (as indicated) for 5 minutes at room temperature. The permeabilisation procedure allows the detection of intracellular localized compartments. Membrane-bound proteins are visualized without permeabilising the cells. Blocking of cells occurred for a minimum of 1 hour in PBS + 1% BSA (USB). Primary antibodies (monoclonal anti-myc, Santa Cruz, sc-40 and polyclonal anti-AS160 Upstate) were incubated overnight. The secondary antibodies used are anti-rabbit Alexa488 to detect isoform2 of AS160 and anti-mouse A546 to detect GLUT4-myc. Incubation occurred for 1 hour (dark). Cells were mounted in 15 µl Dako Cytomation Fluorescent Mounting Medium (DakoCytomation), examined by confocal laser-scanning microscopy with Leica DM IRE2 and analyzed with Leica DM SDK software.

Pictures obtained are shown in Figure 21. The data indicate that isoform2 of AS160 and GLUT4-myc are both localized in the peri-nuclear compartment of cells under basal conditions. Insulin stimulation (100 nM) induces the translocation of GLUT4-myc to the plasma membrane. isoform2 of AS1 60 protein remains in the peri-nuclear compartment.

### REFERENCE LIST

Bouzakri,K. and Zierath,J.R. (2007). MAP4K4 gene silencing in human skeletal muscle prevents TNF-alpha -induced insulin resistance. J Biol Chem. 282, 7783-7789
Bowen,W.P. and Wylie,P.G. (2006). Application of laser-scanning fluorescence microplate cytometry in high content screening. Assay. Drug Dev. Technol. 4, 209-221.
Ciaraldi,T.P., Carter,L., Rehman,N., Mohideen,P., Mudaliar,S., and Henry,R.R. (2002). Insulin and insulin-like growth factor-1 action on human skeletal muscle: preferential effects of insulin-like growth factor-1 in type 2 diabetic subjects. Metabolism. 51, 1171-1179.
DeSilva,D.R., Jones,E.A., Favata,M.F., Jaffee,B.D., Magolda,R.L., Trzaskos,J.M., and Scherle,P.A. (1998). Inhibition of mitogen-activated protein kinase kinase blocks T cell proliferation but does not induce or prevent anergy. J Immunol. 160, 4175-4181.
Elbashir S.M., Harborth J., Lendeckel W., Yalcin A., Weber K., and Tuschl T. (2001). Duplexes of 21-nucleotides RNAs mediate RNA interference in mammalian cell culture. Nature. 411. 494-498
Greene,E.L., Nelson,B.A., Robinson,K.A., and Buse,M.G. (2001). alpha-Lipoic acid prevents the development of glucose-induced insulin resistance in 3T3-L1 adipocytes and accelerates the decline in immunoreactive insulin during cell incubation. Metabolism. 50, 1063-1069.
Hu,Y., Qiao,L., Wang,S., Rong,S.B., Meuillet,E.J., Berggren,M., Gallegos,A., Powis,G., and Kozikowski,A.P. (2000). 3-(Hydroxymethyl)-bearing phosphatidylinositol ether lipid analogues and carbonate surrogates block PI3-K, Akt, and cancer cell growth. J Med Chem. 43, 3045-3051.
Kane,S., Sano,H., Liu,S.C., Asara,J.M., Lane,W.S., Garner,C.C., and Lienhard,G.E. (2002). A method to identify serine kinase substrates. Akt phosphorylates a novel adipocyte protein with a Rab GTPase-activating protein (GAP) domain. J Biol. Chem. 277, 22115-22118.
Karlsson,H.K., Hallsten,K., Bjornholm,M., Tsuchida,H., Chibalin,A.V., Virtanen,K.A., Heinonen,O.J., Lonnqvist,F., Nuutila,P., and Zierath,J.R. (2005a). Effects of metformin and rosiglitazone treatment on insulin signaling and glucose uptake in patients with newly diagnosed type 2 diabetes: a randomized controlled study. Diabetes. 54, 1459-1467.
Karlsson,H.K., Zierath,J.R., Kane,S., Krook,A., Lienhard,G.E., and Wallberg-Henriksson,H. (2005b). Insulin-stimulated phosphorylation of the Akt substrate AS160 is impaired in skeletal muscle of type 2 diabetic subjects. Diabetes. 54, 1692-1697.
Nelson,B.A., Robinson,K.A., and Buse,M.G. (2002). Defective Akt activation is associated with glucose- but not glucosamine-induced insulin resistance. Am. J Physiol Endocrinol Metab. 282, E497-E506.
Okada,T., Kawano,Y., Sakakibara,T., Hazeki,O., and Ui,M. (1994). Essential role of phosphatidylinositol 3-kinase in insulin-induced glucose transport and antilipolysis in rat adipocytes. Studies with a selective inhibitor wortmannin. J Biol. Chem. 269, 3568-3573.
Sano,H., Kane,S., Sano,E., Miinea,C.P., Asara,J.M., Lane,W.S., Garner,C.W., and Lienhard,G.E. (2003). Insulin-stimulated phosphorylation of a Rab GTPase-activating protein regulates GLUT4 translocation. J Biol. Chem. 278, 14599-14602.
Tusch T., Zamore PD., Lehmann R., Bartel DP. and Sharp PA (1999). Targeted mRNA degradation by double-stranded RNA in vitro. Genes and Development 13. 3191-3197
Voss,M.D., Beha,A., Tennagels,N., Tschank,G., Herling,A.W., Quint,M., Gerl,M., Metz-Weidmann,C., Haun,G., and Korn,M. (2005). Gene expression profiling in skeletal muscle of Zucker diabetic fatty rats: implications for a role of stearoyl-CoA desaturase 1 in insulin resistance. Diabetologia. 48, 2622-2630.
Walgren,J.L., Vincent,T.S., Schey,K.L., and Buse,M.G. (2003). High glucose and insulin promote O-GlcNAc modification of proteins, including alpha-tubulin. Am. J Physiol Endocrinol Metab. 284, E424-E434.
Wang Q., Khayat Z., Kishi Y., Ebina Y., and Klip A. (1998). GLUT4 translocation by insulin in intact muscle cells: detection by a fast and quantitative assay. FEBS Lett. 427 (2),193-197
Yuan,J.S., Reed,A., Chen,F., and Stewart,C.N., Jr. (2006). Statistical analysis of real-time PCR data. BMC Bioinformatics. 7:85., 85.
Zeigerer,A., McBrayer,M.K., and McGraw,T.E. (2004). Insulin stimulation of GLUT4 exocytosis, but not its inhibition of endocytosis, is dependent on RabGAP AS160. Mol. Biol. Cell. 15, 4406-4415.

If not indicated otherwise, standard laboratory methods were or can be performed according to standard procedures known in the art, e.g. as outlined the following laboratory standard literature:
Sambrook et al. (1989) Molecular Cloning: A Laboratory Manual. Second edition. Cold Spring Harbor Laboratory Press, Cold Spring Harbor, NY. 545 pp;
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly uptdated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated; Wiley & Sons, Inc; Editors: John E. Coligan, Ben M. Dunn, Hidde L. Ploegh, David W. Speicher, Paul T. Wingfield.
Molecular Biology of the Cell; third edition; Alberts, B., Bray, D., Lewis, J., Raff, M., Roberts, K., Watson, J.D.; Garland Publishing, Inc. New York & London, 1994;
Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York"
Gene targeting: A Practical Approach, 2nd Ed., Joyner AL, ed. 2000. IRL Press at Oxford University Press, New York;

## Claims

1. A method of identifying a substance altering glucose uptake of a cell comprising
(a) contacting a test system comprising AKT substrate 160kDa-like protein (AS160-like protein) with a test substance, and
(b) identifying a test substance as a substance altering glucose uptake and/or GLUT4 translocation to the plasma membrane of a cell by detecting a signal indicative for altered glucose uptake of a cell, wherein the AS160-like protein
(i) is encoded by the nucleic acid having SEQ ID NO: 1,
(ii) is encoded by a nucleic acid encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein,
(iii) is a protein having the amino acid sequence according to SEQ ID NO: 3,
(iv) has 99, 5% or more sequence homology with SEQ ID NO: 3, or
(v) is a protein having an amino acid sequence according to the sequence NM_014832 but lacking the amino acids 678 to 740 when compared to this AS 160 amino acid sequence,
wherein the test system is in a cell of an insulin-dependent tissue.

2. The method of claim 1, wherein glucose uptake of the cell is increased or decreased, preferably increased.

3. The method of claim 1 or 2, wherein the substance alters glucose uptake and/or GLUT4 translocation to the plasma membrane in at least one, two or three insulin-sensitive tissues.

4. The method of claim 3, wherein the insulin-sensitive tissue is adipose tissue, skeletal muscle and/or liver.

5. The method of any of claims 1 to 4, wherein the substance alters glucose uptake and/or GLUT4 translocation to the plasma membrane of a hepatocyte, an adipocyte and/or a skeletal muscle cell.

6. The method of any of claims 1 to 5, wherein the AS 160-like protein is isoform 2 of AS160.

7. The method of any of claims 1 to 6, wherein the AS160-like protein consists of the sequence encoded by SEQ ID NO: 1.

8. The method of any of claims 1 to 7, wherein the test system is in a cell.

9. The method of claim 8, wherein the cell is a skeletal muscle cell, an adipocyte and/or hepatocyte, particularly a skeletal muscle cell.

10. The method of claim 8 or 9, wherein the cell is a cell from a cell line.

11. The method of any of claims 1 to 10, wherein the detectable signal is the amount of AS160-like protein expressed in a cell, phosphorylated AKT, phosphorylated AS160-like protein, GLUT4 translocation to the plasma membrane, GLUT4 distribution in a cell or glucose uptake by a cell.

12. The method of any of claims 1 to 11, wherein the test compound is provided in the form of a chemical compound library.

13. The method of any of the claims 1 to 12, wherein the method is carried out in a robotics system.

14. The method according to any of the claims 1 to 13, wherein the method is a method of high-through put screening.

15. A test system for the identification of a substance for improving glucose uptake and/or GLUT4 translocation to the plasma membrane of a cell, the test system comprising a gene coding for the AKT substrate 160 kDa-like protein (AS160-like protein); and
an inducible promoter providing for controllable expression of the gene,
wherein the activation of AS 160-like protein effects a detectable signal and wherein the AS 160-like protein is defined as in claim 1.

16. The test system of claim 15, wherein the test system is located in a cell, particularly a genetically engineered cell.

17. The test system of claim 16, wherein the gene and/or the promoter is/are introduced into the genetically engineered cell.

18. The test system of any of claims 15 to 16, wherein the inducible promoter is a tetracycline-inducible promoter.

19. In vitro use of a tests system comprising AS160-like protein for the identification of a substance altering, particularly improving, glucose uptake and/or GLUT4 translocation to the plasma membrane of a cell wherein the AS 160-like protein is defined as in claim 1.

20. In vitro use of AS160-like protein thereof in a model for type 2 diabetes wherein the AS 160-like protein is defined as in claim 1.

21. In vitro use of a cell heterologously expressing AS 160-like protein thereof as or in a model for type 2 diabetes wherein the AS160-like protein is defined as in claim 1.

22. The method, use or test system according to any of the preceding claims 7 to 21, wherein the AS 160-like protein is isoform 2 of the AS160 protein.

23. A polypeptide
(i) encoded by the nucleic acid having SEQ ID NO: 1,
(ii) encoded by a nucleic acid encoding a protein consisting of the SEQ ID NO: 3 and short C- and/or N-terminal sequences of at most 30, 25, 20, 15, 10, 9, 8, 7, 6, 5, 4, 3, 2, or 1 amino acids homologous or heterologous to the protein,
(iii) having the amino acid sequence according to SEQ ID NO: 3,
(iv) having 99,5% or more sequence homology with SEQ ID NO: 3 or
(v) having an amino acid sequence according to the sequence NM_014832 but lacking the amino acids 678 to 740 when compared to this AS 160 amino acid sequence.

24. A polynucleotide consisting of the polynucleotide sequence according to SEQ ID NO: 1 or encoding a polypeptide according to SEX ID NO: 3 or encoding a protein consisting of SEQ ID NO: 3 and short C- and/or N-terminal sequences of at most 30, 25, 20, 15, 10, 9, 8, 7, 6, 5, 4, 3, 2, or 1 amino acids homologous or heterologous to the protein.

25. An antibody specifically binding to a polypeptide according to claim 23.

26. A cell heterologously expressing the polypeptide according to claim 23.

27. A cell stably or transiently transfected with a polynucleotide according to claim 24.

## Patentansprüche

1. Verfahren zur Identifizierung einer die Glucose-Aufnahme einer Zelle verändernden Substanz, bei dem man
(a) ein AKT-Substrat 160 kDa ähnliches Protein (AS160 ähnliches Protein) umfassendes Testsystem mit einer Testsubstanz in Kontakt bringt und
(b) eine Testsubstanz als eine die Glucose-Aufnahme und/oder GLUT4-Translokation an die Plasmamembran einer Zelle verändernde Substanz durch Nachweisen eines Signals, das auf eine veränderte Glucose-Aufnahme einer Zelle hindeutet, identifiziert, wobei das AS160 ähnliche Protein
(i) durch die Nukleinsäure mit der SEQ ID NO: 1 codiert ist,
(ii) durch eine für ein aus der SEQ ID NO: 3 und kurzen C- und/oder N-terminalen Sequenzen von höchstens 30, 25, 20, 15, 10, 9, 8, 7, 6, 5, 4, 3, 2 oder 1 Aminosäuren, die homolog oder heterolog zu dem Protein sind, bestehendes Protein codierende Nukleinsäure codiert ist,
(iii) ein Protein mit der Aminosäuresequenz gemäß SEQ ID NO: 3 ist,
(iv) 99,5% oder mehr Sequenzhomologie mit der SEQ ID NO: 3 aufweist oder
(v) ein eine Aminosäuresequenz gemäß der Sequenz NM_014832 aufweisendes Protein ist, jedoch verglichen mit dieser AS-160-Aminosäuresequenz ohne die Aminosäuren 678 bis 740;
wobei sich das Testsystem in einer Zelle eines insulinabhängigen Gewebes befindet.

2. Verfahren nach Anspruch 1, wobei die Glucose-Aufnahme der Zelle erhöht oder reduziert, vorzugsweise erhöht ist.

3. Verfahren nach Anspruch 1 oder 2, wobei die Substanz die Glucose-Aufnahme und/oder GLUT4-Translokation an die Plasmamembran in wenigstens einem, zwei oder drei insulinempfindlichen Geweben verändert.

4. Verfahren nach Anspruch 3, wobei es sich bei dem insulinempfindlichen Gewebe um Fettgewebe, Skelettmuskel und/oder Leber handelt.

5. Verfahren nach einem der Ansprüche 1 bis 4, wobei die Substanz die Glucose-Aufnahme und/oder GLUT4-Translokation an die Plasmamembran eines Hepatozyten, eines Adipozyten und/oder einer Skelettmuskelzelle verändert.

6. Verfahren nach einem der Ansprüche 1 bis 5, wobei es sich bei dem AS160 ähnlichen Protein um Isoform 2 von AS160 handelt.

7. Verfahren nach einem der Ansprüche 1 bis 6, wobei das AS160 ähnliche Protein die durch SEQ ID NO: 1 codierte Sequenz besteht.

8. Verfahren nach einem der Ansprüche 1 bis 7, wobei sich das Testsystem in einer Zelle befindet.

9. Verfahren nach Anspruch 8, wobei es sich bei der Zelle um eine Skelettmuskelzelle, einen Adipozyten und/oder Hepatozyten, insbesondere eine Skelettmuskelzelle handelt.

10. Verfahren nach Anspruch 8 oder 9, wobei es sich bei der Zelle um eine Zelle aus einer Zelllinie handelt.

11. Verfahren nach einem der Ansprüche 1 bis 10, wobei es sich bei dem nachweisbaren Signal um die Menge an in einer Zelle exprimiertem AS160 ähnlichem Protein, phosphoryliertes AKT, phosphoryliertes AS160 ähnliches Protein, GLUT4-Translokation an die Plasmamembran, GLUT4-Verteilung in einer Zelle oder Glucose-Aufnahme durch eine Zelle handelt.

12. Verfahren nach einem der Ansprüche 1 bis 11, wobei die Testverbindung in Form einer Bibliothek chemischer Verbindungen bereitgestellt wird.

13. Verfahren nach einem der Ansprüche 1 bis 12, wobei das Verfahren in einem Robotiksystem durchgeführt wird.

14. Verfahren nach einem der Ansprüche 1 bis 13, wobei es sich bei dem Verfahren um ein Screening-Verfahren mit hohem Durchsatz handelt.

15. Testsystem zur Identifizierung einer Substanz zur Verbesserung der Glucose-Aufnahme und/oder GLUT4-Translokation an die Plasmamembran einer Zelle, wobei das Testsystem ein für das AKT-Substrat 160 kDa ähnliche Protein (AS160 ähnliche Protein) codierendes Gen; sowie einen für eine steuerbare Expression des Gens sorgenden induzierbaren Promotor umfasst, wobei die Aktivierung des AS160 ähnlichen Proteins ein nachweisbares Signal hervorruft und wobei das AS160 ähnliche Protein die in Anspruch 1 angegebene Bedeutung hat.

16. Testsystem nach Anspruch 15, wobei das Testsystem in einer Zelle, insbesondere einer gentechnisch manipulierten Zelle lokalisiert ist.

17. Testsystem nach Anspruch 16, wobei das Gen und/oder der Promotor in die gentechnisch manipulierte Zelle eingeführt werden bzw. wird.

18. Testsystem nach einem der Ansprüche 15 bis 16, wobei es sich bei dem induzierbaren Promotor um einen durch Tetracyclin induzierbaren Promotor handelt.

19. In-vitro-Verwendung eines AS160 ähnliches Protein umfassenden Testsystems zur Identifizierung einer die Glucose-Aufnahme und/oder GLUT4-Translokation an die Plasmamembran einer Zelle verändernden, insbesondere verbessernden Substanz, wobei das AS160 ähnliche Protein die in Anspruch 1 angegebene Bedeutung hat.

20. In-vitro-Verwendung von AS160 ähnlichem Protein davon in einem Modell für Typ-2-Diabetes, wobei das AS160 ähnliche Protein die in Anspruch 1 angegebene Bedeutung hat.

21. In-vitro-Verwendung einer AS160 ähnliches Protein davon heterolog exprimierenden Zelle als oder in einem Modell für Typ-2-Diabetes, wobei das AS160 ähnliche Protein die in Anspruch 1 angegebene Bedeutung hat.

22. Verfahren, Verwendung oder Testsystem nach einem der vorhergehenden Ansprüche 7 bis 21, wobei es sich bei dem AS160 ähnlichen Protein um Isoform 2 von AS160-Protein handelt.

23. Polypeptid,
(i) codiert durch die Nukleinsäure mit der SEQ ID NO: 1,
(ii) codiert durch eine für ein aus der SEQ ID NO: 3 und kurzen C- und/oder N-terminalen Sequenzen von höchstens 30, 25, 20, 15, 10, 9, 8, 7, 6, 5, 4, 3, 2 oder 1 Aminosäuren, die homolog oder heterolog zu dem Protein sind, bestehendes Protein codierende Nukleinsäure,
(iii) mit der Aminosäuresequenz gemäß SEQ ID NO: 3,
(iv) aufweisend 99,5% oder mehr Sequenzhomologie mit der SEQ ID NO: 3, oder
(v) aufweisend eine Aminosäuresequenz gemäß der Sequenz NM_014832, jedoch verglichen mit dieser AS-160-Aminosäuresequenz ohne die Aminosäuren 678 bis 740.

24. Polynukleotid, bestehend aus der Polynukleotidsequenz gemäß SEQ ID NO: 1 oder codierend für ein Polypeptid gemäß SEQ ID NO: 3 oder codierend für ein Protein, das aus SEQ ID NO: 3 und kurzen C- und/oder N-terminalen Sequenzen von höchstens 30, 25, 20, 15, 10, 9, 8, 7, 6, 5, 4, 3, 2 oder 1 Aminosäuren, die homolog oder heterolog zu dem Protein sind, besteht.

25. Antikörper mit spezifischer Bindung an ein Polypeptid gemäß Anspruch 23.

26. Zelle mit heterologer Expression des Polypeptids gemäß Anspruch 23.

27. Zelle, stabil oder transient transfiziert mit einem Polynukleotid gemäß Anspruch 24.

## Revendications

1. Procédé d'identification d'une substance altérant l'absorption du glucose d'une cellule, comprenant les étapes consistant à :
(a) mettre en contact un système de test, comprenant une protéine de type substrat de l'AKT de 160 kDa (protéine de type AS160), avec une substance à tester et
(b) identifier une substance à tester comme étant une substance qui altère l'absorption du glucose et/ou la translocation du GLUT4 vers la membrane plasmique d'une cellule, par la détection d'un signal indicatif d'une absorption altérée du glucose d'une cellule, dans lequel la protéine de type AS160
(i) est codée par l'acide nucléique ayant la SEQ ID n° : 1,
(ii) est codée par un acide nucléique codant une protéine constituée par la SEQ ID n° : 3 et des séquences courtes C- et/ou N-terminales d'au plus 30, 25, 20, 15, 10, 9, 8, 7, 6, 5, 4, 3, 2 ou 1 acide(s) aminé(s) homologue(s) ou hétérologue(s) à la protéine,
(iii) est une protéine ayant la séquence d'acides aminés selon la SEQ ID n° : 3,
(iv) a une homologie de séquence de 99,5 % ou plus avec la SEQ ID n° : 3
ou
(v) est une protéine ayant une séquence d'acides aminés selon la séquence NM_014832 mais dépourvue des acides aminés 678 à 740 lorsqu'elle est comparée à cette séquence d'acides aminés de l'AS160,
dans lequel le système de test se trouve dans une cellule d'un tissu dépendant de l'insuline.

2. Procédé selon la revendication 1, dans lequel l'absorption du glucose de la cellule est accrue ou diminuée, de préférence accrue.

3. Procédé selon la revendication 1 ou la 2, dans lequel la substance altère l'absorption du glucose et/ou la translocation du GLUT4 vers la membrane plasmique dans au moins un, deux ou trois tissu(s) sensible(s) à l'insuline.

4. Procédé selon la revendication 3, dans lequel le tissu sensible à l'insuline est le tissu adipeux, un muscle squelettique et/ou le foie.

5. Procédé selon l'une quelconque des revendications 1 à 4, dans lequel la substance altère l'absorption du glucose et/ou la translocation du GLUT4 vers la membrane plasmique d'un hépatocyte, d'un adipocyte et/ou d'une cellule de muscle squelettique.

6. Procédé selon l'une quelconque des revendications 1 à 5, dans lequel la protéine de type AS160 est l'isoforme 2 de l'AS160.

7. Procédé selon l'une quelconque des revendications 1 à 6, dans lequel la protéine de type AS160 est constituée de la séquence codée par la SEQ ID n° : 1.

8. Procédé selon l'une quelconque des revendications 1 à 7, dans lequel le système de test se trouve dans une cellule.

9. Procédé selon la revendication 8, dans lequel la cellule est une cellule de muscle squelettique, un adipocyte et/ou un hépatocyte, en particulier une cellule de muscle squelettique.

10. Procédé selon la revendication 8 ou la 9, dans lequel la cellule est une cellule provenant d'une lignée de cellules.

11. Procédé selon l'une quelconque des revendications 1 à 10, dans lequel le signal détectable est la quantité de protéine de type AS160 exprimée dans une cellule, d'AKT phosphorylée, de protéine de type AS160 phosphorylée, de translocation du GLUT4 vers la membrane plasmique, de distribution du GLUT4 dans une cellule ou d'absorption du glucose par une cellule.

12. Procédé selon l'une quelconque des revendications 1 à 11, dans lequel le composé à tester est fourni sous la forme d'une banque de composés chimiques.

13. Procédé selon l'une quelconque des revendications 1 à 12, dans lequel le procédé est exécuté dans un système robotisé.

14. Procédé selon l'une quelconque des revendications 1 à 13, dans lequel le procédé est un procédé de criblage à haut débit.

15. Système de test pour l'identification d'une substance destinée à améliorer l'absorption du glucose et/ou la translocation du GLUT4 vers la membrane plasmique d'une cellule, le système de test comprenant un gène codant pour la protéine de type substrat de l'AKT de 160 kDa (protéine de type AS160) ; et
un promoteur inductible assurant une expression contrôlable du gène, dans lequel l'activation de la protéine de type AS160 génère un signal détectable et
dans lequel la protéine de type AS160 est définie tel que dans la revendication 1.

16. Système de test selon la revendication 15, dans lequel le système de test est situé dans une cellule, en particulier une cellule obtenue par génie génétique.

17. Système de test selon la revendication 16, dans lequel le gène et/ou le promoteur est/sont introduit(s) dans la cellule obtenue par génie génétique.

18. Système de test selon l'une quelconque des revendications 15 à 16, dans lequel le promoteur inductible est un promoteur inductible par la tétracycline.

19. Utilisation *in vitro* d'un système de test comprenant une protéine de type AS160 pour l'identification d'une substance altérant, en particulier améliorant, l'absorption du glucose et/ou la translocation du GLUT4 vers la membrane plasmique d'une cellule, dans lequel la protéine de type AS160 est définie tel que dans la revendication 1.

20. Utilisation *in vitro* d'une protéine de type AS160 de celle-ci dans un modèle destiné au diabète de type 2, dans lequel la protéine de type AS160 est définie tel que dans la revendication 1.

21. Utilisation *in vitro* d'une cellule exprimant, de manière hétérologue, une protéine de type AS160 de celle-ci comme modèle pour le diabète de type 2 ou dans celui-ci, dans lequel la protéine de type AS160 est définie telle que dans la revendication 1.

22. Procédé, utilisation ou système de test selon l'une quelconque des revendications 7 à 21 précédentes, dans laquelle la protéine de type AS160 est l'isoforme 2 de la protéine AS160.

23. Polypeptide :
(i) codé par l'acide nucléique ayant la SEQ ID n° : 1,
(ii) codé par un acide nucléique codant une protéine constituée par la SEQ ID n° : 3 et des séquences courtes C- et/ou N-terminales d'au plus 30, 25, 20, 15, 10, 9, 8, 7, 6, 5, 4, 3, 2 ou 1 acide(s) aminé(s) homologue(s) ou hétérologue(s) à la protéine,
(iii) ayant la séquence d'acides aminés selon la SEQ ID n° : 3,
(iv) ayant une homologie de séquence de 99,5 % ou plus avec la SEQ ID n° : 3 ou
(v) ayant une séquence d'acides aminés selon la séquence NM_014832 mais dépourvue des acides aminés 678 à 740 lorsqu'elle est comparée à cette séquence d'acides aminés de l'AS160.

24. Polynucléotide constitué par la séquence de polynucléotides selon la SEQ ID n° : 1 ou codant pour un polypeptide selon la SEQ ID n° : 3 ou codant pour une protéine constituée par la SEQ ID n° : 3 et des séquences courtes C- et/ou N-terminales d'au plus 30, 25, 20, 15, 10, 9, 8, 7, 6, 5, 4, 3, 2 ou 1 acide(s) aminé(s) homologue(s) ou hétérologue(s) à la protéine.

25. Anticorps se liant spécifiquement à un polypeptide selon la revendication 23.

26. Cellule exprimant, de manière hétérologue, le polypeptide selon la revendication 23.

27. Cellule transfectée, de manière stable ou transitoire, avec un polynucléotide selon la revendication 24.


### SEQUENCE LISTING

<110> sanofi-aventis
<120> Novel AS160-like protein, test systems, methods and uses involving it for the identification of diabetes type 2 therapeutics
<130> DE2007/034
<150> 07215086.7
   <151> 2007-08-01
<160> 23
<170> PatentIn version 3.3
<210> 1
   <211> 3707
   <212> DNA
   <213> Homo sapiens
<400> 1
   atggagccgc ccagctgcat tcaggatgag ccgttcccgc accccctgga gcccgagccg      60
   ggcgtctcag ctcagcccgg ccccgggaag ccaagcgata agcggttccg gctgtggtac     120
   gttggggggt cgtgcctgga ccacaggacc acgctgccta tgctgccctg gctcatggcc      180
   gagatccgca ggcgcagcca gaagcccgag gcgggcggct gcggggcgcc ggcggcccga     240
   gaggtgatcc tggtgctcag cgcgcccttc ctgcgttgcg tccccgcgcc gggcgctggg     300
   gcctcggggg gcactagtcc gtcggccacg cagcccaacc cggcggtatt catcttcgag     360
   cacaaggcgc agcatatctc gcgcttcatc cacaacagcc acgacctcac ctactttgcc     420
   tacctgatca aggcgcagcc cgacgacccc gagtcgcaga tggcctgcca cgttttccgc     480
   gccacagacc ccagccaggt tcctgatgtt attagcagca taaggcaatt atctaaagcg     540
   gccatgaaag aggatgccaa acccagcaaa gataatgagg acgcctttta caactctcag     600
   aagtttgaag tcctgtactg tggaaaggtg accgtgaccc acaagaaggc cccctcaagc     660
   ctcatcgatg actgcatgga gaagttcagc ctgcacgaac agcagcgcct gaagatccaa     720
   ggcgagcagc gcggtccgga cccaggagag gacctggctg acttggaggt ggtggtgccc     780
   gggtcccccg gagactgcct gccggaggag gctgacggca ccgacaccca ccttggctta     840
   cctgccgggg ccagccagcc tgccctgacc agctctcggg tctgcttccc tgagcggatt     900
   ttggaagatt ctggctttga tgagcagcag gagtttcggt ctcggtgcag cagtgtcacc     960
   ggcgtgcaac ggagagttca cgagggcagc cagaaatccc agccgcgacg gagacacgcg    1020
   agcgcaccca gtcacgtcca gccctcggac tcggagaaga acaggaccat gctcttccag    1080
   gttgggcgat ttgagattaa ccttatcagt ccagacacta aatcagttgt gctagaaaag    1140
   aattttaaag atatctcctc ttgttctcag ggtataaagc atgtggatca ctttggcttt    1200
   atctgccggg agtctccaga gcctggactt agccagtata tttgttatgt attccagtgt    1260
   gccagcgaat ctctggttga tgaggtaatg ctgactctga aacaggcctt cagtacggcg    1320
   gctgccctgc agagtgccaa gacgcagatt aaactgtgtg aggcctgccc gatgcactct    1380
   ttgcataagc tctgtgaaag gattgaaggt ctctacccac caagagccaa gctggtgata   1440
   cagaggcatc tctcatcact gacagataat gagcaagctg acatctttga aagagttcag   1500
   aaaatgaagc cagtcagtga ccaggaagaa aatgaacttg tgattttaca cctgaggcag   1560
   ctgtgtgaag ccaagcagaa gacacacgtg cacatcgggg aaggcccttc tactatttca   1620
   aatagtacaa tcccagaaaa tgcaacaagc agtggaaggt tcaaacttga cattctgaaa   1680
   aataaagcta agagatcctt aactagctcc ctggaaaata tcttctcaag gggagctaac   1740
   agaatgagag gtcggcttgg aagtgtggac agttttgaac ggtccaacag tcttgcttca   1800
   gagaaggact actcaccagg ggattctcca ccagggacac cgccagcgtc cccaccgtcc   1860
   tcagcttggc aaacgtttcc cgaagaggat tccgactccc cgcagtttcg aagacgggca   1920
   cacacgttca gccacccacc ttcaagcaca aagagaaagc tgaatttgca ggatgggagg   1980
   gctcagggtg tgcgttcccc tctgctgagg cagagctcca gtgaacagtg cagtgatgga   2040
   gaagggagaa aaaggacctc atctacctgc agcaatgagt ccctaagtgt gggaggaacc   2100
   tctgtcactc ctcgccggat ctcctggcgg cagcgcattt tcctcagggt tgcttctccc   2160
   atgaacaaat ctccctcagc aatgcaacag caagatggat tggacaggaa cgagctgctg   2220
   ccactgtccc ccctctctcc aaccatggag gaggaaccgc tggttatatt cctgtctggg   2280
   gaggatgacc cagaaaagat tgaagaaaga aagaaatcaa aagaactgag gagcttgtgg   2340
   agaaaagcta tacaccaaca aatcttgtta cttcgaatgg aaaaagaaaa ccagaaactt   2400
   gaaggagcaa gcagagatga actccagtcc agaaaagtta aattagacta tgaagaagtt   2460
   ggtgcatgtc agaaagaggt cttaataact tgggataaga agttgttaaa ctgcagagct   2520
   aaaatcagat gtgatatgga agatattcat actcttctta aagaaggagt tcccaaaagt   2580
   cgacgaggag aaatttggca gtttctggct ttacagtacc gactcagaca cagattgcct   2640
   aataaacaac agcctcctga catatcctat aaggaacttt tgaagcagct cactgctcag   2700
   cagcatgcga ttctcgtgga tttaggaagg acgtttccta ctcaccctta cttttcagta   2760
   cagcttgggc caggacagct gtcactgttt aacctcctga aagcctattc tttgctggac   2820
   aaagaagtgg gatactgtca ggggatcagc tttgtggctg gagtcctgct tctgcacatg   2880
   agtgaagagc aagcctttga aatgctgaaa ttcctcatgt atgacctcgg cttccgcaag   2940
   cagtacagac ctgacatgat gtcgctgcag attcaaatgt accagctgtc caggctcctt   3000
   catgactatc acagagatct ctacaatcac cttgaagaaa atgaaatcag ccccagtctt   3060
   tatgctgccc cctggttcct cacattgttt gcctctcagt tttcattagg atttgtagcc   3120
   agagtttttg atattatttt tcttcaggga actgaagtta tattcaaggt tgcactcagc   3180
   ctactgagca gccaagagac acttataatg gaatgtgaga gctttgaaaa tattgttgag   3240
   tttcttaaaa acacgctacc tgatatgaat acctctgaaa tggaaaaaat tattacccag   3300
   gtttttgaga tggatatttc taagcagttg catgcctatg aggtggaata tcatgtgcta   3360
   caggatgagc ttcaggaatc ttcatattcc tgtgaggata gtgaaacttt ggagaagctg   3420
   gagagggcca atagccaact gaaaagacaa aacatggacc tcctagaaaa attacaggta 3480
   gctcatacta aaatccaggc cttggaatca aacctggaaa atcttttgac gagagagacc 3540
   aaaatgaagt ctttaatccg gaccctggaa caagaaaaaa tggcttatca aaagacagtg 3600
   gagcaactcc ggaagctgct gcccgcggat gctctagtca attgtgacct gttgctgaga 3660
   gacctaaact gcaaccctaa caacaaagcc aagataggaa ataagcc 3707
<210> 2
   <211> 5818
   <212> DNA
   <213> Artificial
<220>
   <223> Vector pIRES-puro2/TetR
<400> 2
   gacggatcgg gagatctccc gatcccctat ggtcgactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggac tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa attaatacga ctcactatag ggagacccaa gcttggtacc    900
   gagctcggat cgatatctgc ggcctagcta gcgtttaaac ttaagcttac catgtctaga    960
   ttagataaaa gtaaagtgat taacagcgca ttagagctgc ttaatgaggt cggaatcgaa   1020
   ggtttaacaa cccgtaaact cgcccagaag ctaggtgtag agcagcctac attgtattgg   1080
   catgtaaaaa ataagcgggc tttgctcgac gccttagcca ttgagatgtt agataggcac   1140
   catactcact tttgcccttt agaaggggaa agctggcaag attttttacg taataacgct   1200
   aaaagtttta gatgtgcttt actaagtcat cgcgatggag caaaagtaca tttaggtaca   1260
   cggcctacag aaaaacagta tgaaactctc gaaaatcaat tagccttttt atgccaacaa   1320
   ggtttttcac tagagaatgc attatatgca ctcagcgctg tggggcattt tactttaggt   1380
   tgcgtattgg aagatcaaga gcatcaagtc gctaaagaag aaagggaaac acctactact   1440
   gatagtatgc cgccattatt acgacaagct atcgaattat ttgatcacca aggtgcagag 1500
   ccagccttct tattcggcct tgaattgatc atatgcggat tagaaaaaca acttaaatgt 1560
   gaaagtgggt ccgcgtacag cggatcccgg gaattcagat cttattaagc ggccgcatag 1620
   ataactgatc cagtgtgctg gaattaattc gctgtctgcg agggccagct gttggggtga 1680
   gtactccctc tcaaaagcgg gcatgacttc tgcgctaaga ttgtcagttt ccaaaaacga 1740
   ggaggatttg atattcacct ggcccgcggt gatgcctttg agggtggccg cgtccatctg 1800
   gtcagaaaag acaatctttt tgttgtcaag cttgaggtgt ggcaggcttg agatctggcc 1860
   atacacttga gtgacaatga catccacttt gcctttctct ccacaggtgt ccactcccag 1920
   gtccaactgc aggtcgagca tgcatctagg gcggccaatt ccgcccctct ccctcccccc 1980
   cccctaacgt tactggccga agccgcttgg aataaggccg gtgtgcgttt gtctatatgt 2040
   gattttccac catattgccg tcttttggca atgtgagggc ccggaaacct ggccctgtct 2100
   tcttgacgag cattcctagg ggtctttccc ctctcgccaa aggaatgcaa ggtctgttga 2160
   atgtcgtgaa ggaagcagtt cctctggaag cttcttgaag acaaacaacg tctgtagcga 2220
   ccctttgcag gcagcggaac cccccacctg gcgacaggtg cctctgcggc caaaagccac 2280
   gtgtataaga tacacctgca aaggcggcac aaccccagtg ccacgttgtg agttggatag 2340
   ttgtggaaag agtcaaatgg ctctcctcaa gcgtattcaa caaggggctg aaggatgccc 2400
   agaaggtacc ccattgtatg ggatctgatc tggggcctcg gtgcacatgc tttacatgtg 2460
   tttagtcgag gttaaaaaaa cgtctaggcc ccccgaacca cggggacgtg gttttccttt 2520
   gaaaaacacg atgataagct tgccacaacc cacaaggaga cgaccttcca tgaccgagta 2580
   caagcccacg gtgcgcctcg ccacccgcga cgacgtcccc cgggccgtac gcaccctcgc 2640
   cgccgcgttc gccgactacc ccgccacgcg ccacaccgtc gacccggacc gccacatcga 2700
   gcgggtcacc gagctgcaag aactcttcct cacgcgcgtc gggctcgaca tcggcaaggt 2760
   gtgggtcgcg gacgacggcg ccgcggtggc ggtctggacc acgccggaga gcgtcgaagc 2820
   gggggcggtg ttcgccgaga tcggcccgcg catggccgag ttgagcggtt cccggctggc 2880
   cgcgcagcaa cagatggaag gcctcctggc gccgcaccgg cccaaggagc ccgcgtggtt 2940
   cctggccacc gtcggcgtct cgcccgacca ccagggcaag ggtctgggca gcgccgtcgt 3000
   gctccccgga gtggaggcgg ccgagcgcgc cggggtgccc gccttcctgg agacctccgc 3060
   gccccgcaac ctccccttct acgagcggct cggcttcacc gtcaccgccg acgtcgagtg 3120
   cccgaaggac cgcgcgacct ggtgcatgac ccgcaagccc ggtgcctgac gcccgcccca 3180
   cgacccgcag cgcccgaccg aaaggagcgc acgaccccat ggctccgacc gaagccgacc 3240
   cgggcggccc cgccgacccc gcacccgccc ccgaggccca ccgactctag agctcgctga 3300
   tcagcctcga ctgtgccttc tagttgccag ccatctgttg tttgcccctc ccccgtgcct 3360
   tccttgaccc tggaaggtgc cactcccact gtcctttcct aataaaatga ggaaattgca 3420
   tcgcattgtc tgagtaggtg tcattctatt ctggggggtg gggtggggca ggacagcaag 3480
   ggggaggatt gggaagacaa tagcaggcat gctggggatg cggtgggctc tatggcttct 3540
   gaggcggaaa gaaccagctg gggctcgagt gcattctagt tgtggtttgt ccaaactcat 3600
   caatgtatct tatcatgtct gtataccgtc gacctctagc tagagcttgg cgtaatcatg 3660
   gtcatagctg tttcctgtgt gaaattgtta tccgctcaca attccacaca acatacgagc 3720
   cggaagcata aagtgtaaag cctggggtgc ctaatgagtg agctaactca cattaattgc 3780
   gttgcgctca ctgcccgctt tccagtcggg aaacctgtcg tgccagctgc attaatgaat 3840
   cggccaacgc gcggggagag gcggtttgcg tattgggcgc tcttccgctt cctcgctcac 3900
   tgactcgctg cgctcggtcg ttcggctgcg gcgagcggta tcagctcact caaaggcggt 3960
   aatacggtta tccacagaat caggggataa cgcaggaaag aacatgtgag caaaaggcca 4020
   gcaaaaggcc aggaaccgta aaaaggccgc gttgctggcg tttttccata ggctccgccc 4080
   ccctgacgag catcacaaaa atcgacgctc aagtcagagg tggcgaaacc cgacaggact 4140
   ataaagatac caggcgtttc cccctggaag ctccctcgtg cgctctcctg ttccgaccct 4200
   gccgcttacc ggatacctgt ccgcctttct cccttcggga agcgtggcgc tttctcaatg 4260
   ctcacgctgt aggtatctca gttcggtgta ggtcgttcgc tccaagctgg gctgtgtgca 4320
   cgaacccccc gttcagcccg accgctgcgc cttatccggt aactatcgtc ttgagtccaa 4380
   cccggtaaga cacgacttat cgccactggc agcagccact ggtaacagga ttagcagagc 4440
   gaggtatgta ggcggtgcta cagagttctt gaagtggtgg cctaactacg gctacactag 4500
   aaggacagta tttggtatct gcgctctgct gaagccagtt accttcggaa aaagagttgg 4560
   tagctcttga tccggcaaac aaaccaccgc tggtagcggt ggtttttttg tttgcaagca 4620
   gcagattacg cgcagaaaaa aaggatctca agaagatcct ttgatctttt ctacggggtc 4680
   tgacgctcag tggaacgaaa actcacgtta agggattttg gtcatgagat tatcaaaaag 4740
   gatcttcacc tagatccttt taaattaaaa atgaagtttt aaatcaatct aaagtatata 4800
   tgagtaaact tggtctgaca gttaccaatg cttaatcagt gaggcaccta tctcagcgat 4860
   ctgtctattt cgttcatcca tagttgcctg actccccgtc gtgtagataa ctacgatacg 4920
   ggagggctta ccatctggcc ccagtgctgc aatgataccg cgagacccac gctcaccggc 4980
   tccagattta tcagcaataa accagccagc cggaagggcc gagcgcagaa gtggtcctgc 5040
   aactttatcc gcctccatcc agtctattaa ttgttgccgg gaagctagag taagtagttc 5100
   gccagttaat agtttgcgca acgttgttgc cattgctaca ggcatcgtgg tgtcacgctc 5160
   gtcgtttggt atggcttcat tcagctccgg ttcccaacga tcaaggcgag ttacatgatc 5220
   ccccatgttg tgcaaaaaag cggttagctc cttcggtcct ccgatcgttg tcagaagtaa 5280
   gttggccgca gtgttatcac tcatggttat ggcagcactg cataattctc ttactgtcat 5340
   gccatccgta agatgctttt ctgtgactgg tgagtactca accaagtcat tctgagaata 5400
   gtgtatgcgg cgaccgagtt gctcttgccc ggcgtcaata cgggataata ccgcgccaca 5460
   tagcagaact ttaaaagtgc tcatcattgg aaaacgttct tcggggcgaa aactctcaag 5520
   gatcttaccg ctgttgagat ccagttcgat gtaacccact cgtgcaccca actgatcttc 5580
   agcatctttt actttcacca gcgtttctgg gtgagcaaaa acaggaaggc aaaatgccgc 5640
   aaaaaaggga ataagggcga cacggaaatg ttgaatactc atactcttcc tttttcaata 5700
   ttattgaagc atttatcagg gttattgtct catgagcgga tacatatttg aatgtattta 5760
   gaaaaataaa caaatagggg ttccgcgcac atttccccga aaagtgccac ctgacgtc 5818
<210> 3
   <211> 1236
   <212> PRT
   <213> Homo sapiens
<400> 3
   Met Glu Pro Pro Ser Cys Ile Gln Asp Glu Pro Phe Pro His Pro Leu
   1 5 10 15
   Glu Pro Glu Pro Gly Val Ser Ala Gln Pro Gly Pro Gly Lys Pro Ser
   20 25 30
   Asp Lys Arg Phe Arg Leu Trp Tyr Val Gly Gly Ser Cys Leu Asp His
   35 40 45
   Arg Thr Thr Leu Pro Met Leu Pro Trp Leu Met Ala Glu Ile Arg Arg
   50 55 60
   Arg Ser Gln Lys Pro Glu Ala Gly Gly Cys Gly Ala Pro Ala Ala Arg
   65 70 75 80
   Glu Val Ile Leu Val Leu Ser Ala Pro Phe Leu Arg Cys Val Pro Ala
   85 90 95
   Pro Gly Ala Gly Ala Ser Gly Gly Thr Ser Pro Ser Ala Thr Gln Pro
   100 105 110
   Asn Pro Ala Val Phe Ile Phe Glu His Lys Ala Gln His Ile Ser Arg
   115 120 125
   Phe Ile His Asn Ser His Asp Leu Thr Tyr Phe Ala Tyr Leu Ile Lys
   130 135 140
   Ala Gln Pro Asp Asp Pro Glu Ser Gln Met Ala Cys His Val Phe Arg
   145 150 155 160
   Ala Thr Asp Pro Ser Gln Val Pro Asp Val Ile Ser Ser Ile Arg Gln
   165 170 175
   Leu Ser Lys Ala Ala Met Lys Glu Asp Ala Lys Pro Ser Lys Asp Asn
   180 185 190
   Glu Asp Ala Phe Tyr Asn Ser Gln Lys Phe Glu Val Leu Tyr Cys Gly
   195 200 205
   Lys Val Thr Val Thr His Lys Lys Ala Pro Ser Ser Leu Ile Asp Asp
   210 215 220
   Cys Met Glu Lys Phe Ser Leu His Glu Gln Gln Arg Leu Lys Ile Gln
   225 230 235 240
   Gly Glu Gln Arg Gly Pro Asp Pro Gly Glu Asp Leu Ala Asp Leu Glu
   245 250 255
   Val Val Val Pro Gly Ser Pro Gly Asp Cys Leu Pro Glu Glu Ala Asp
   260 265 270
   Gly Thr Asp Thr His Leu Gly Leu Pro Ala Gly Ala Ser Gln Pro Ala
   275 280 285
   Leu Thr Ser Ser Arg Val Cys Phe Pro Glu Arg Ile Leu Glu Asp Ser
   290 295 300
   Gly Phe Asp Glu Gln Gln Glu Phe Arg Ser Arg Cys Ser Ser Val Thr
   305 310 315 320
   Gly Val Gln Arg Arg Val His Glu Gly Ser Gln Lys Ser Gln Pro Arg
   325 330 335
   Arg Arg His Ala Ser Ala Pro Ser His Val Gln Pro Ser Asp Ser Glu
   340 345 350
   Lys Asn Arg Thr Met Leu Phe Gln Val Gly Arg Phe Glu Ile Asn Leu
   355 360 365
   Ile Ser Pro Asp Thr Lys Ser Val Val Leu Glu Lys Asn Phe Lys Asp
   370 375 380
   Ile Ser Ser Cys Ser Gln Gly Ile Lys His Val Asp His Phe Gly Phe
   385 390 395 400
   Ile Cys Arg Glu Ser Pro Glu Pro Gly Leu Ser Gln Tyr Ile Cys Tyr
   405 410 415
   Val Phe Gln Cys Ala Ser Glu Ser Leu Val Asp Glu Val Met Leu Thr
   420 425 430
   Leu Lys Gln Ala Phe Ser Thr Ala Ala Ala Leu Gln Ser Ala Lys Thr
   435 440 445
   Gln Ile Lys Leu Cys Glu Ala Cys Pro Met His Ser Leu His Lys Leu
   450 455 460
   Cys Glu Arg Ile Glu Gly Leu Tyr Pro Pro Arg Ala Lys Leu Val Ile
   465                 470                 475                 480
   Gln Arg His Leu Ser Ser Leu Thr Asp Asn Glu Gln Ala Asp Ile Phe
                   485                 490                 495
   Glu Arg Val Gln Lys Met Lys Pro Val Ser Asp Gln Glu Glu Asn Glu
               500                 505                 510
   Leu Val Ile Leu His Leu Arg Gln Leu Cys Glu Ala Lys Gln Lys Thr
           515                 520                 525
   His Val His Ile Gly Glu Gly Pro Ser Thr Ile Ser Asn Ser Thr Ile
       530                 535                 540
   Pro Glu Asn Ala Thr Ser Ser Gly Arg Phe Lys Leu Asp Ile Leu Lys
   545                 550                 555                 560
   Asn Lys Ala Lys Arg Ser Leu Thr Ser Ser Leu Glu Asn Ile Phe Ser
                   565                 570                 575
   Arg Gly Ala Asn Arg Met Arg Gly Arg Leu Gly Ser Val Asp Ser Phe
               580                 585                 590
   Glu Arg Ser Asn Ser Leu Ala Ser Glu Lys Asp Tyr Ser Pro Gly Asp
           595                 600                 605
   Ser Pro Pro Gly Thr Pro Pro Ala Ser Pro Pro Ser Ser Ala Trp Gln
       610                 615                 620
   Thr Phe Pro Glu Glu Asp Ser Asp Ser Pro Gln Phe Arg Arg Arg Ala
   625                 630                 635                 640
   His Thr Phe Ser His Pro Pro Ser Ser Thr Lys Arg Lys Leu Asn Leu
                   645                 650                 655
   Gln Asp Gly Arg Ala Gln Gly Val Arg Ser Pro Leu Leu Arg Gln Ser
               660                 665                 670
   Ser Ser Glu Gln Cys Ser Asp Gly Glu Gly Arg Lys Arg Thr Ser Ser
           675                 680                 685
   Thr Cys Ser Asn Glu Ser Leu Ser Val Gly Gly Thr Ser Val Thr Pro
       690                 695                 700
   Arg Arg Ile Ser Trp Arg Gln Arg Ile Phe Leu Arg Val Ala Ser Pro
   705                 710                 715                 720
   Met Asn Lys Ser Pro Ser Ala Met Gln Gln Gln Asp Gly Leu Asp Arg
                   725                 730                 735
   Asn Glu Leu Leu Pro Leu Ser Pro Leu Ser Pro Thr Met Glu Glu Glu
   740 745 750
   Pro Leu Val Ile Phe Leu Ser Gly Glu Asp Asp Pro Glu Lys Ile Glu
   755 760 765
   Glu Arg Lys Lys Ser Lys Glu Leu Arg Ser Leu Trp Arg Lys Ala Ile
   770 775 780
   His Gln Gln Ile Leu Leu Leu Arg Met Glu Lys Glu Asn Gln Lys Leu
   785 790 795 800
   Glu Gly Ala Ser Arg Asp Glu Leu Gln Ser Arg Lys Val Lys Leu Asp
   805 810 815
   Tyr Glu Glu Val Gly Ala Cys Gln Lys Glu Val Leu Ile Thr Trp Asp
   820 825 830
   Lys Lys Leu Leu Asn Cys Arg Ala Lys Ile Arg Cys Asp Met Glu Asp
   835 840 845
   Ile His Thr Leu Leu Lys Glu Gly Val Pro Lys Ser Arg Arg Gly Glu
   850 855 860
   Ile Trp Gln Phe Leu Ala Leu Gln Tyr Arg Leu Arg His Arg Leu Pro
   865 870 875 880
   Asn Lys Gln Gln Pro Pro Asp Ile Ser Tyr Lys Glu Leu Leu Lys Gln
   885 890 895
   Leu Thr Ala Gln Gln His Ala Ile Leu Val Asp Leu Gly Arg Thr Phe
   900 905 910
   Pro Thr His Pro Tyr Phe Ser Val Gln Leu Gly Pro Gly Gln Leu Ser
   915 920 925
   Leu Phe Asn Leu Leu Lys Ala Tyr Ser Leu Leu Asp Lys Glu Val Gly
   930 935 940
   Tyr Cys Gln Gly Ile Ser Phe Val Ala Gly Val Leu Leu Leu His Met
   945 950 955 960
   Ser Glu Glu Gln Ala Phe Glu Met Leu Lys Phe Leu Met Tyr Asp Leu
   965 970 975
   Gly Phe Arg Lys Gln Tyr Arg Pro Asp Met Met Ser Leu Gln Ile Gln
   980 985 990
   Met Tyr Gln Leu Ser Arg Leu Leu His Asp Tyr His Arg Asp Leu Tyr
   995 1000 1005
   Asn His Leu Glu Glu Asn Glu Ile Ser Pro Ser Leu Tyr Ala Ala
   1010 1015 1020
   Pro Trp Phe Leu Thr Leu Phe Ala Ser Gln Phe Ser Leu Gly Phe
   1025 1030 1035
   Val Ala Arg Val Phe Asp Ile Ile Phe Leu Gln Gly Thr Glu Val
   1040 1045 1050
   Ile Phe Lys Val Ala Leu Ser Leu Leu Ser Ser Gln Glu Thr Leu
   1055 1060 1065
   Ile Met Glu Cys Glu Ser Phe Glu Asn Ile Val Glu Phe Leu Lys
   1070 1075 1080
   Asn Thr Leu Pro Asp Met Asn Thr Ser Glu Met Glu Lys Ile Ile
   1085 1090 1095
   Thr Gln Val Phe Glu Met Asp Ile Ser Lys Gln Leu His Ala Tyr
   1100 1105 1110
   Glu Val Glu Tyr His Val Leu Gln Asp Glu Leu Gln Glu Ser Ser
   1115 1120 1125
   Tyr Ser Cys Glu Asp Ser Glu Thr Leu Glu Lys Leu Glu Arg Ala
   1130 1135 1140
   Asn Ser Gln Leu Lys Arg Gln Asn Met Asp Leu Leu Glu Lys Leu
   1145 1150 1155
   Gln Val Ala His Thr Lys Ile Gln Ala Leu Glu Ser Asn Leu Glu
   1160 1165 1170
   Asn Leu Leu Thr Arg Glu Thr Lys Met Lys Ser Leu Ile Arg Thr
   1175 1180 1185
   Leu Glu Gln Glu Lys Met Ala Tyr Gln Lys Thr Val Glu Gln Leu
   1190 1195 1200
   Arg Lys Leu Leu Pro Ala Asp Ala Leu Val Asn Cys Asp Leu Leu
   1205 1210 1215
   Leu Arg Asp Leu Asn Cys Asn Pro Asn Asn Lys Ala Lys Ile Gly
   1220 1225 1230
   Asn Lys Pro
   1235
<210> 4
   <211> 28
   <212> DNA
   <213> Artificial
<220>
   <223> Primer/Probe
<400> 4
   catactcttc ttaaagaagg agttccca 28
<210> 5
   <211> 26
   <212> DNA
   <213> Artificial
<220>
   <223> Primer/Probe
<400> 5
   ctgtgtctga gtcggtactg taaagc 26
<210> 6
   <211> 30
   <212> DNA
   <213> Artificial
<220>
   <223> Primer/Probe
<400> 6
   cagaaactgc caaatttctc ctcgtcgact 30
<210> 7
   <211> 18
   <212> DNA
   <213> Artificial
<220>
   <223> Primer/Probe
<400> 7
   gcgttcccct ctgctgag 18
<210> 8
   <211> 23
   <212> DNA
   <213> Artificial
<220>
   <223> Primer/Probe
<400> 8
   actcattgct gcaggtagat gag 23
<210> 9
   <211> 30
   <212> DNA
   <213> Artificial
<220>
   <223> primer/probe
<400> 9
   ttctccatca ctgcactgtt cactggagct 30
<210> 10
   <211> 23
   <212> DNA
   <213> Artificial
<220>
   <223> Primer/Probe
<400> 10
   acagcggaag tggtattgta cgt 23
<210> 11
   <211> 19
   <212> DNA
   <213> Artificial
<220>
   <223> Primer/Probe
<400> 11
   ggcactgtgg ttcctgcat 19
<210> 12
   <211> 22
   <212> DNA
   <213> Artificial
<220>
   <223> Primer/Probe
<400> 12
   caggcaccgc cagccactgt ct 22
<210> 13
   <211> 5055
   <212> DNA
   <213> Homo sapiens
<400> 13
   gcggccgcgg ggaccctcgg cgtggtcctc tgaccctgca aacccgcgac ggaggaaggg 60
   gaggtcctgc ccgaggcgcc agcccaagga ggaggatgcc catttaaccc gccctcgcct 120
   gccgggcgct tgcctcggtg cccgccgccg gagcctccga gccgcgcccg tggaagtgct 180
   gcatggggca gggctgctga agcgcggagt tcggggtcgc gccgctccca ggcaggcgcg 240
   ggagcccggt gcggcagttg gcacagtttc ggcggcgcct tctgcgcggg agtggggggc 300
   gcggtgcgcc cggccggcct ccgcggtgcc ctggtgaggc gagagttatg gagccgccca 360
   gctgcattca ggatgagccg ttcccgcacc ccctggagcc cgagccgggc gtctcagctc 420
   agcccggccc cgggaagcca agcgataagc ggttccggct gtggtacgtt ggggggtcgt 480
   gcctggacca caggaccacg ctgcctatgc tgccctggct catggccgag atccgcaggc 540
   gcagccagaa gcccgaggcg ggcggctgcg gggcgccggc ggcccgagag gtgatcctgg 600
   tgctcagcgc gcccttcctg cgttgcgtcc ccgcgccggg cgctggggcc tcggggggca 660
   ctagtccgtc ggccacgcag cccaacccgg cggtattcat cttcgagcac aaggcgcagc 720
   atatctcgcg cttcatccac aacagccacg acctcaccta ctttgcctac ctgatcaagg 780
   cgcagcccga cgaccccgag tcgcagatgg cctgccacgt tttccgcgcc acagacccca 840
   gccaggttcc tgatgttatt agcagcataa ggcaattatc taaagcggcc atgaaagagg 900
   atgccaaacc cagcaaagat aatgaggacg ccttttacaa ctctcagaag tttgaagtcc 960
   tgtactgtgg aaaggtgacc gtgacccaca agaaggcccc ctcaagcctc atcgatgact 1020
   gcatggagaa gttcagcctg cacgaacagc agcgcctgaa gatccaaggc gagcagcgcg 1080
   gtccggaccc aggagaggac ctggctgact tggaggtggt ggtgcccggg tcccccggag 1140
   actgcctgcc ggaggaggct gacggcaccg acacccacct tggcttacct gccggggcca 1200
   gccagcctgc cctgaccagc tctcgggtct gcttccctga gcggattttg gaagattctg 1260
   gctttgatga gcagcaggag tttcggtctc ggtgcagcag tgtcaccggc gtgcaacgga 1320
   gagttcacga gggcagccag aaatcccagc cgcgacggag acacgcgagc gcacccagtc 1380
   acgtccagcc ctcggactcg gagaagaaca ggaccatgct cttccaggtt gggcgatttg 1440
   agattaacct tatcagtcca gacactaaat cagttgtgct agaaaagaat tttaaagata 1500
   tctcctcttg ttctcagggt ataaagcatg tggatcactt tggctttatc tgccgggagt 1560
   ctccagagcc tggacttagc cagtatattt gttatgtatt ccagtgtgcc agcgaatctc 1620
   tggttgatga ggtaatgctg actctgaaac aggccttcag tacggcggct gccctgcaga 1680
   gtgccaagac gcagattaaa ctgtgtgagg cctgccccat gcactctttg cataagctct 1740
   gtgaaaggat tgaaggtctc tacccaccaa gagccaagct ggtgatacag aggcatctct 1800
   catcactgac agataatgag caagctgaca tctttgaaag agttcagaaa atgaagccag 1860
   tcagtgacca ggaagaaaat gaacttgtga ttttacacct gaggcagctg tgtgaagcca 1920
   agcagaagac acacgtgcac atcggggaag gcccttctac tatttcaaat agtacaatcc 1980
   cagaaaatgc aacaagcagt ggaaggttca aacttgacat tctgaaaaat aaagctaaga 2040
   gatccttaac tagctccctg gaaaatatct tctcaagggg agctaacaga atgagaggtc 2100
   ggcttggaag tgtggacagt tttgaacggt ccaacagtct tgcttcagag aaggactact 2160
   caccagggga ttctccacca gggacaccgc cagcgtcccc accgtcctca gcttggcaaa 2220
   cgtttcccga agaggattcc gactccccgc agtttcgaag acgggcacac acgttcagcc 2280
   acccaccttc aagcacaaag agaaagctga atttgcagga tgggagggct cagggtgtgc 2340
   gttcccctct gctgaggcag agctccagtg aacagtgcag tgatggagaa gggagaaaaa 2400
   ggacctcatc tacctgcagc aatgagtccc taagtgtggg aggaacctct gtcactcctc 2460
   gccggatctc ctggcggcag cgcattttcc tcagggttgc ttctcccatg aacaaatctc 2520
   cctcagcaat gcaacagcaa gatggattgg acaggaacga gctgctgcca ctgtcccccc 2580
   tctctccaac catggaggag gaaccgctgg ttatattcct gtctggggag gatgacccag 2640
   aaaagattga agaaagaaag aaatcaaaag aactgaggag cttgtggaga aaagctatac 2700
   accaacaaat cttgttactt cgaatggaaa aagaaaacca gaaacttgaa ggagcaagca 2760
   gagatgaact ccagtccaga aaagttaaat tagactatga agaagttggt gcatgtcaga 2820
   aagaggtctt aataacttgg gataagaagt tgttaaactg cagagctaaa atcagatgtg 2880
   atatggaaga tattcatact cttcttaaag aaggagttcc caaaagtcga cgaggagaaa 2940
   tttggcagtt tctggcttta cagtaccgac tcagacacag attgcctaat aaacaacagc 3000
   ctcctgacat atcctataag gaacttttga agcagctcac tgctcagcag catgcgattc 3060
   tcgtggattt aggaaggacg tttcctactc acccttactt ttcagtacag cttgggccag 3120
   gacagctgtc actgtttaac ctcctgaaag cctattcttt gctggacaaa gaagtgggat 3180
   actgtcaggg gatcagcttt gtggctggag tcctgcttct gcacatgagt gaagagcaag 3240
   cctttgaaat gctgaaattc ctcatgtatg acctcggctt ccgcaagcag tacagacctg 3300
   acatgatgtc gctgcagatt caaatgtacc agctgtccag gctccttcat gactatcaca 3360
   gagatctcta caatcacctt gaagaaaatg aaatcagccc cagtctttat gctgccccct 3420
   ggttcctcac attgtttgcc tctcagtttt cattaggatt tgtagccaga gtttttgata 3480
   ttatttttct tcagggaact gaagttatat tcaaggttgc actcagccta ctgagcagcc 3540
   aagagacact tataatggaa tgtgagagct ttgaaaatat tgttgagttt cttaaaaaca 3600
   cgctacctga tatgaatacc tctgaaatgg aaaaaattat tacccaggtt tttgagatgg 3660
   atatttctaa gcagttgcat gcctatgagg tggaatatca tgtgctacag gatgagcttc 3720
   aggaatcttc atattcctgt gaggatagtg aaactttgga gaagctggag agggccaata 3780
   gccaactgaa aagacaaaac atggacctcc tagaaaaatt acaggtagct catactaaaa 3840
   tccaggcctt ggaatcaaac ctggaaaatc ttttgacgag agagaccaaa atgaagtctt 3900
   taatccggac cctggaacaa gaaaaaatgg cttatcaaaa gacagtggag caactccgga 3960
   agctgctgcc cgcggatgct ctagtcaatt gtgacctgtt gctgagagac ctaaactgca 4020
   accctaacaa caaagccaag ataggaaata agccccaggt ttttgagatg gatatttcta 4080
   agcagttgca tgcctatgag gtggaatatc atgtgctaca ggatgagctt caggaatctt 4140
   catattcctg tgaggatagt gaaactttgg agaagctgga gagggccaat agccaactga 4200
   aaagacaaaa catggacctc ctagaaaaat tacaggtagc tcatactaaa atccaggcct 4260
   tggaatcaaa cctggaaaat cttttgacga gagagaccaa aatgaagtct ttaatccgga 4320
   ccctggaaca agaaaaaatg gcttatcaaa agacagtgga gcaactccgg aagctgctgc 4380
   ccgcggatgc tctagtcaat tgtgacctgt tgctgagaga cctaaactgc aaccctaaca 4440
   acaaagccaa gataggaaat aagccataat tgaagaggca cggcctcagc agaaagtgct 4500
   ccttagaata ctacagagag gaagagcctg catgtcgctg gcccaaggct ggaccctgaa 4560
   gctgatggaa ccacctaata ctggtgctga gcgcctagtc acagcaggtg gacctcgtgc 4620
   tcatcagagc atgccaatcc taagccattg gacatatgta gactggtttt tgttgttgct 4680
   atgtacatat aaatatatat ataaaatgaa catagttcat gctttcagat aaaatgagta 4740
   gatgtatatt tagattaatt tttttagtca gaacttcatg aaatccacac caaaggaaag 4800
   gtaaactgaa atttcccttg gacatatgtg aaatcttttt gtctttatag tgaaacaaag 4860
   ccagagcatc tttgtatatt gcaatatact tgaaaaaaat gaatgtattt ttttctccaa 4920
   agaacagcat gtttcactca atggtgaaaa ggtggaaaca tttatgtaac tttatgtgta 4980
   tctgtcttga tatctactga cattgtctat atgaggaaaa tgattactgg tcatgctcct 5040
   gtgagttttt tggga 5055
<210> 14
   <211> 21
   <212> DNA
   <213> Artificial
<220>
   <223> Primer/Probe
<400> 14
   ggaggaggat gcccatttaa c 21
<210> 15
   <211> 23
   <212> DNA
   <213> Artificial
<220>
   <223> Primer/Probe
<400> 15
   tctaaggagc actttctgct gag 23
<210> 16
   <211> 28
   <212> DNA
   <213> Artificial
<220>
   <223> Primer/Probe
<400> 16
   agcttttacc agaattcagg tagactgt 28
<210> 17
   <211> 22
   <212> DNA
   <213> Artificial
<220>
   <223> Primer/Probe
<400> 17
   tgctgcaggt agatgaggtc ct 22
<210> 18
   <211> 22
   <212> DNA
   <213> Artificial
<220>
   <223> Primer/Probe
<400> 18
   cttctccatc actgatttca tt 22
<210> 19
   <211> 26
   <212> DNA
   <213> Artificial
<220>
   <223> Primer/Probe
<400> 19
   attcaggtag actgtcccca cagtat 26
<210> 20
   <211> 25
   <212> DNA
   <213> Artificial
<220>
   <223> Primer/Probe
<400> 20
   ccttctccat cacttgattc tgaag 25
<210> 21
   <211> 21
   <212> DNA
   <213> Artificial
<220>
   <223> Primer/Probe
<400> 21
   atgaaatcag acaagacact g 21
<210> 22
   <211> 3881
   <212> DNA
   <213> Homo sapiens
<400> 22
   atggagccgc ccagctgcat tcaggatgag ccgttcccgc accccctgga gcccgagccg     60
   ggcgtctcag ctcagcccgg ccccgggaag ccaagcgata agcggttccg gctgtggtac    120
   gttggggggt cgtgcctgga ccacaggacc acgctgccta tgctgccctg gctcatggcc    180
   gagatccgca ggcgcagcca gaagcccgag gcgggcggct gcggggcgcc ggcggcccga    240
   gaggtgatcc tggtgctcag cgcgcccttc ctgcgttgcg tccccgcgcc gggcgctggg    300
   gcctcggggg gcactagtcc gtcggccacg cagcccaacc cggcggtatt catcttcgag    360
   cacaaggcgc agcatatctc gcgcttcatc cacaacagcc acgacctcac ctactttgcc    420
   tacctgatca aggcgcagcc cgacgacccc gagtcgcaga tggcctgcca cgttttccgc    480
   gccacagacc ccagccaggt tcctgatgtt attagcagca taaggcaatt atctaaagcg    540
   gccatgaaag aggatgccaa acccagcaaa gataatgagg acgcctttta caactctcag    600
   aagttcgaag tcctgtactg tggaaaggtg accgtgaccc acaagaaggc cccctcaagc    660
   ctcatcgatg actgcatgga gaagttcagc ctgcacgaac agcagcgcct gaagatccaa    720
   ggggagcagc gcggtccgga cccaggagag gacctggctg acttggaggt ggtggtgccc    780
   gggtcccccg gagactgcct gccggaggag gctgacggca ccgacaccca ccttggctta 840
   cctgccgggg ccagccagcc tgccctgacc agctctcggg tctgcttccc tgagcggatt 900
   ttggaagatt ctggctttga tgagcagcag gagtttcggt ctcggtgcag cagtgtcacc 960
   ggcgtgcaac ggagagttca cgagggcagc cagaaatccc agccgcgacg gagacacgcg 1020
   agcgcaccca gtcacgtcca gccctcggac tcggagaaga acaggaccat gctcttccag 1080
   gttgggcgat ttgagattaa ccttatcagt ccagacacta aatcagttgt gctagaaaag 1140
   aattttaaag atatctcctc ttgttctcag ggtataaagc atgtggatca ctttggcttt 1200
   atctgccggg agtctccaga gcctggactt agccagtata tttgttatgt attccagtgt 1260
   gccagcgaat ctctggttga tgaggtaatg ctgactctga aacaggcctt cagtacggcg 1320
   gctgccctgc agagtgccaa gacgcagatt aaactgtgtg aggcctgccc gatgcactct 1380
   ttgcataagc tctgtgaaag gattgaaggt ctctacccac caagagccaa gctggtgata 1440
   cagaggcatc tctcatcact gacagataat gagcaagctg acatctttga aagagttcag 1500
   aaaatgaagc cagtcagtga ccaggaagaa aatgaacttg tgattttaca cctgaggcag 1560
   ctgtgtgaag ccaagcagaa gacacacgtg cacatcgggg aaggcccttc tactatttca 1620
   aatagtacaa tcccagaaaa tgcaacaagc agtggaaggt tcaaacttga cattctgaaa 1680
   aataaagcta agagatcctt aactagctcc ctggaaaata tcttctcaag gggagctaac 1740
   agaatgagag gtcggcttgg aagtgtggac agttttgaac ggtccaacag tcttgcttca 1800
   gagaaggact actcaccagg ggattctcca ccagggacac cgccagcgtc cccaccgtcc 1860
   tcagcttggc aaacgtttcc cgaagaggat tccgactccc cgcagtttcg aagacgggca 1920
   cacacgttca gccacccacc ttcaagcaca aagagaaagc tgaatttgca ggatgggagg 1980
   gctcagggtg tgcgttcccc tctgctgagg cagagctcca gtgaacagtg cagcaatctt 2040
   tcgtcagttc gacgcatgta caaggagagt aattcttcct ccagtcttcc aagtcttcac 2100
   acttccttct ctgccccttc cttcactgcc ccctctttcc tgaaaagctt ttaccagaat 2160
   tcaggtagac tgtccccaca gtatgaaaat gaaatcagac tgatggagaa gggagaaaaa 2220
   ggacctcatc tacctgcagc aatgagtccc taagtgtggg aggaacctct gtcactcctc 2280
   gccggatctc ctggcggcag cgcattttcc tcagggttgc ttctcccatg aacaaatctc 2340
   cctcagcaat gcaacagcaa gatggattgg acaggaacga gctgctgcca ctgtcccccc 2400
   tctctccaac catggaggag gaaccgctgg ttgtattcct gtctggggag gatgacccag 2460
   aaaagattga agaaagaaag aaatcaaaag aactgaggag cttgtggaga aaagctatac 2520
   accaacaaat cttgttactt cgaatggaaa aagaaaacca gaaacttgaa gcaagcagag 2580
   atgaactcca gtccagaaaa gttaaattag actatgaaga agttggtgca tgtcagaaag 2640
   aggtcttaat aacttgggat aagaagttgt taaactgcag agctaaaatc agatgtgata 2700
   tggaagatat tcatactctt cttaaagaag gagttcccaa aagtcgacga ggagaaattt 2760
   ggcagtttct ggctttacag taccgactca gacacagatt gcctaataaa caacagcctc 2820
   ctgacatatc ctataaggaa cttttgaagc agctcactgc tcagcagcat gcgattctcg    2880
   tggatttagg aaggacgttt cctactcacc cttacttttc agtacagctt gggccaggac    2940
   agctgtcact gtttaacctc ctgaaagcct attctttgct ggacaaagaa gtgggatact    3000
   gtcaggggat cagctttgtg gctggagtcc tgcttctgca catgagtgaa gagcaagcct    3060
   ttgaaatgct gaaattcctc atgtatgacc tcggcttccg caagcagtac agacctgaca    3120
   tgatgtcgct gcagattcaa atgtaccagc tgtccaggct ccttcatgac tatcacagag    3180
   atctctacaa tcaccttgaa gaaaatgaaa tcagccccag tctttatgct gccccctggt    3240
   tcctcacatt gtttgcctct cagttttcat taggatttgt agccagagtt tttgatatta    3300
   tttttcttca gggaactgaa gttatattca aggttgcact cagcctactg agcagccaag    3360
   agacacttat aatggaatgt gagagctttg aaaatattgt tgagtttctt aaaaacacgc    3420
   tacctgatat gaatacctct gaaatggaaa aaattattac ccaggttttt gagatggata    3480
   tttctaagca gttgcatgcc tatgaggtgg aatatcatgt gctacaggat gagcttcagg    3540
   aatcttcata ttcctgtgag gatagtgaaa ctttggagaa gctggagagg gccaatagcc    3600
   aactgaaaag acaaaacatg gacctcctag aaaaattaca ggtagctcat actaaaatcc    3660
   aggccttgga atcaaacctg gaaaatcttt tgacgagaga gaccaaaatg aagtctttaa    3720
   tccggaccct ggaacaagaa aaaatggctt atcaaaagac agtggagcaa ctccggaagc    3780
   tgctgcccgc ggatgctcta gtcaattgtg acctgttgct gagagaccta aactgcaacc    3840
   ctaacaacaa agccaagata ggaaataagc cataattgaa g                        3881
<210> 23
   <211> 1264
   <212> PRT
   <213> Homo sapiens
<400> 23
   Met Glu Pro Pro Ser Cys Ile Gln Asp Glu Pro Phe Pro His Pro Leu
   1               5                   10                  15
   Glu Pro Glu Pro Gly Val Ser Ala Gln Pro Gly Pro Gly Lys Pro Ser
               20                  25                  30
   Asp Lys Arg Phe Arg Leu Trp Tyr Val Gly Gly Ser Cys Leu Asp His
           35                  40                  45
   Arg Thr Thr Leu Pro Met Leu Pro Trp Leu Met Ala Glu Ile Arg Arg
       50                  55                  60
   Arg Ser Gln Lys Pro Glu Ala Gly Gly Cys Gly Ala Pro Ala Ala Arg
   65                  70                  75                  80
   Glu Val Ile Leu Val Leu Ser Ala Pro Phe Leu Arg Cys Val Pro Ala
                   85                  90                  95
   Pro Gly Ala Gly Ala Ser Gly Gly Thr Ser Pro Ser Ala Thr Gln Pro
               100                 105                 110
   Asn Pro Ala Val Phe Ile Phe Glu His Lys Ala Gln His Ile Ser Arg
           115                 120                 125
   Phe Ile His Asn Ser His Asp Leu Thr Tyr Phe Ala Tyr Leu Ile Lys
       130                 135                 140
   Ala Gln Pro Asp Asp Pro Glu Ser Gln Met Ala Cys His Val Phe Arg
   145                 150                 155                 160
   Ala Thr Asp Pro Ser Gln Val Pro Asp Val Ile Ser Ser Ile Arg Gln
                   165                 170                 175
   Leu Ser Lys Ala Ala Met Lys Glu Asp Ala Lys Pro Ser Lys Asp Asn
               180                 185                 190
   Glu Asp Ala Phe Tyr Asn Ser Gln Lys Phe Glu Val Leu Tyr Cys Gly
           195                 200                 205
   Lys Val Thr Val Thr His Lys Lys Ala Pro Ser Ser Leu Ile Asp Asp
       210                 215                 220
   Cys Met Glu Lys Phe Ser Leu His Glu Gln Gln Arg Leu Lys Ile Gln
   225                 230                 235                 240
   Gly Glu Gln Arg Gly Pro Asp Pro Gly Glu Asp Leu Ala Asp Leu Glu
                   245                 250                 255
   Val Val Val Pro Gly Ser Pro Gly Asp Cys Leu Pro Glu Glu Ala Asp
               260                 265                 270
   Gly Thr Asp Thr His Leu Gly Leu Pro Ala Gly Ala Ser Gln Pro Ala
           275                 280                 285
   Leu Thr Ser Ser Arg Val Cys Phe Pro Glu Arg Ile Leu Glu Asp Ser
       290                 295                 300
   Gly Phe Asp Glu Gln Gln Glu Phe Arg Ser Arg Cys Ser Ser Val Thr
   305                 310                 315                 320
   Gly Val Gln Arg Arg Val His Glu Gly Ser Gln Lys Ser Gln Pro Arg
                   325                 330                 335
   Arg Arg His Ala Ser Ala Pro Ser His Val Gln Pro Ser Asp Ser Glu
               340                 345                 350
   Lys Asn Arg Thr Met Leu Phe Gln Val Gly Arg Phe Glu Ile Asn Leu
           355                 360                 365
   Ile Ser Pro Asp Thr Lys Ser Val Val Leu Glu Lys Asn Phe Lys Asp
   370 375 380
   Ile Ser Ser Cys Ser Gln Gly Ile Lys His Val Asp His Phe Gly Phe
   385 390 395 400
   Ile Cys Arg Glu Ser Pro Glu Pro Gly Leu Ser Gln Tyr Ile Cys Tyr
   405 410 415
   Val Phe Gln Cys Ala Ser Glu Ser Leu Val Asp Glu Val Met Leu Thr
   420 425 430
   Leu Lys Gln Ala Phe Ser Thr Ala Ala Ala Leu Gln Ser Ala Lys Thr
   435 440 445
   Gln Ile Lys Leu Cys Glu Ala Cys Pro Met His Ser Leu His Lys Leu
   450 455 460
   Cys Glu Arg Ile Glu Gly Leu Tyr Pro Pro Arg Ala Lys Leu Val Ile
   465 470 475 480
   Gln Arg His Leu Ser Ser Leu Thr Asp Asn Glu Gln Ala Asp Ile Phe
   485 490 495
   Glu Arg Val Gln Lys Met Lys Pro Val Ser Asp Gln Glu Glu Asn Glu
   500 505 510
   Leu Val Ile Leu His Leu Arg Gln Leu Cys Glu Ala Lys Gln Lys Thr
   515 520 525
   His Val His Ile Gly Glu Gly Pro Ser Thr Ile Ser Asn Ser Thr Ile
   530 535 540
   Pro Glu Asn Ala Thr Ser Ser Gly Arg Phe Lys Leu Asp Ile Leu Lys
   545 550 555 560
   Asn Lys Ala Lys Arg Ser Leu Thr Ser Ser Leu Glu Asn Ile Phe Ser
   565 570 575
   Arg Gly Ala Asn Arg Met Arg Gly Arg Leu Gly Ser Val Asp Ser Phe
   580 585 590
   Glu Arg Ser Asn Ser Leu Ala Ser Glu Lys Asp Tyr Ser Pro Gly Asp
   595 600 605
   Ser Pro Pro Gly Thr Pro Pro Ala Ser Pro Pro Ser Ser Ala Trp Gln
   610 615 620
   Thr Phe Pro Glu Glu Asp Ser Asp Ser Pro Gln Phe Arg Arg Arg Ala
   625 630 635 640
   His Thr Phe Ser His Pro Pro Ser Ser Thr Lys Arg Lys Leu Asn Leu
                   645                 650                 655
   Gln Asp Gly Arg Ala Gln Gly Val Arg Ser Pro Leu Leu Arg Gln Ser
               660                 665                 670
   Ser Ser Glu Gln Cys Ser Asn Leu Ser Ser Val Arg Arg Met Tyr Lys
           675                 680                 685
   Glu Ser Asn Ser Ser Ser Ser Leu Pro Ser Leu His Thr Ser Phe Ser
       690                 695                 700
   Ala Pro Ser Phe Thr Ala Pro Ser Phe Leu Lys Ser Phe Tyr Gln Asn
   705                 710                 715                 720
   Ser Gly Arg Leu Ser Pro Gln Tyr Glu Asn Glu Ile Arg Leu Met Glu
                   725                 730                 735
   Lys Gly Glu Lys Gly Pro His Leu Pro Ala Ala Met Ser Pro Val Trp
               740                 745                 750
   Glu Glu Pro Leu Ser Leu Leu Ala Gly Ser Pro Gly Gly Ser Ala Phe
           755                 760                 765
   Ser Ser Gly Leu Leu Leu Pro Thr Asn Leu Pro Gln Gln Cys Asn Ser
       770                 775                 780
   Lys Met Asp Trp Thr Gly Thr Ser Cys Cys His Cys Pro Pro Ser Leu
   785                 790                 795                 800
   Gln Pro Trp Arg Arg Asn Arg Trp Leu Tyr Ser Cys Leu Gly Arg Met
                   805                 810                 815
   Thr Gln Lys Arg Leu Lys Lys Glu Arg Asn Gln Lys Asn Gly Ala Cys
               820                 825                 830
   Gly Glu Lys Leu Tyr Thr Asn Lys Ser Cys Tyr Phe Glu Trp Lys Lys
           835                 840                 845
   Lys Thr Arg Asn Leu Lys Gln Ala Glu Met Asn Ser Ser Pro Glu Lys
       850                 855                 860
   Leu Asn Thr Met Lys Lys Leu Val His Val Arg Lys Arg Ser Leu Gly
   865                 870                 875                 880
   Ile Arg Ser Cys Thr Ala Glu Leu Lys Ser Asp Val Ile Trp Lys Ile
                   885                 890                 895
   Phe Ile Leu Phe Leu Lys Lys Glu Phe Pro Lys Val Asp Glu Glu Lys
               900                 905                 910
   Phe Gly Ser Phe Trp Leu Tyr Ser Thr Asp Ser Asp Thr Asp Cys Leu
   915 920 925
   Ile Asn Asn Ser Leu Leu Thr Tyr Pro Ile Arg Asn Phe Ser Ser Ser
   930 935 940
   Leu Leu Ser Ser Met Arg Phe Ser Trp Ile Glu Gly Arg Phe Leu Leu
   945 950 955 960
   Thr Leu Thr Phe Gln Tyr Ser Leu Gly Gln Asp Ser Cys His Cys Leu
   965 970 975
   Thr Ser Lys Pro Ile Leu Cys Trp Thr Lys Lys Trp Asp Thr Val Arg
   980 985 990
   Gly Ser Ala Leu Trp Leu Glu Ser Cys Phe Cys Thr Val Lys Ser Lys
   995 1000 1005
   Pro Leu Lys Cys Asn Ser Ser Cys Met Thr Ser Ala Ser Ala Ser
   1010 1015 1020
   Ser Thr Asp Leu Thr Cys Arg Cys Arg Phe Lys Cys Thr Ser Cys
   1025 1030 1035
   Pro Gly Ser Phe Met Thr Ile Thr Glu Ile Ser Thr Ile Thr Leu
   1040 1045 1050
   Lys Lys Met Lys Ser Ala Pro Val Phe Met Leu Pro Pro Gly Ser
   1055 1060 1065
   Ser His Cys Leu Pro Leu Ser Phe His Asp Leu Pro Glu Phe Leu
   1070 1075 1080
   Ile Leu Phe Phe Phe Arg Glu Leu Lys Leu Tyr Ser Arg Leu His
   1085 1090 1095
   Ser Ala Tyr Ala Ala Lys Arg His Leu Trp Asn Val Arg Ala Leu
   1100 1105 1110
   Lys Ile Leu Leu Ser Phe Leu Lys Thr Arg Tyr Leu Ile Ile Pro
   1115 1120 1125
   Leu Lys Trp Lys Lys Leu Leu Pro Arg Phe Leu Arg Trp Ile Phe
   1130 1135 1140
   Leu Ser Ser Cys Met Pro Met Arg Trp Asn Ile Met Cys Tyr Arg
   1145 1150 1155
   Met Ser Phe Arg Asn Leu His Ile Pro Val Arg Ile Val Lys Leu
   1160 1165 1170
   Trp Arg  Ser Trp Arg Gly Pro  Ile Ala Asn Lys Asp  Lys Thr Trp
       1175                 1180                 1185
   Thr Ser  Lys Asn Tyr Arg Leu  Ile Leu Lys Ser Arg  Pro Trp Asn
       1190                 1195                 1200
   Gln Thr  Trp Lys Ile Phe Arg  Glu Arg Pro Lys Ser  Leu Ser Gly
       1205                 1210                 1215
   Pro Trp  Asn Lys Lys Lys Trp  Leu Ile Lys Arg Gln  Trp Ser Asn
       1220                 1225                 1230
   Ser Gly  Ser Cys Cys Pro Arg  Met Leu Ser Ile Val  Thr Cys Cys
       1235                 1240                 1245
   Glu Thr  Thr Ala Thr Leu Thr  Thr Lys Pro Arg Glu  Ile Ser His
       1250                 1255                 1260
   Asn